# EUROPEAN PATENT APPLICATION

(11) **EP 4 197 550 A1**
(43) Date of publication of application: **21.06.2023**
(21) Application number: 21856308.8
(22) Date of filing: 17.08.2021
(51) Int. Cl.: A61K 38/16, A61K 47/68, A61K 38/17, A61K 38/26, A61P 3/00, A61P 1/16, A61P 9/12, A61K 38/22, A61K 47/60

(54) **HYPOTENSIVE PHARMACEUTICAL COMPOSITION COMPRISING TRIPLE ACTIVATOR HAVING ACTIVITY FOR ALL OF GLUCAGON, GLP-1, AND GIP RECEPTORS**

(30) Priority: 14.08.2020 KR 20200102604; 13.04.2021 KR 20210048006
(71) Applicant: Hanmi Pharm. Co., Ltd., Hwaseong-si, Gyeonggi-do 18536 (KR)
(72) Inventor: BAEK, Seungjae, Seoul 05545 (KR); CHOI, Jaeduk, Seoul 05545 (KR); SHIN, Wonjung, Seoul 05545 (KR); KIM, Jung Kuk, Hwaseong-si, Gyeonggi-do 18469 (KR); LEE, Jong Suk, Hwaseong-si, Gyeonggi-do 18469 (KR); CHOI, Jae Hyuk, Hwaseong-si, Gyeonggi-do 18469 (KR); OH, Euh Lim, Hwaseong-si, Gyeonggi-do 18469 (KR)
(74) Representative: Mewburn Ellis LLP
(86) International application number: PCT/KR2021/010910
(87) International publication number: WO 2022/035302

(57) **Abstract**

The present invention relates to a peptide having activity for all of glucagon, GLP-1, and GIP receptors, and a use thereof.

## Description

### [Technical Field]

The present invention relates to a composition containing a peptide having activities for all of glucagon, GLP-1, and GIP receptors, and use thereof.

### [Background Art]

Glucagon is produced and secreted by the pancreas in response to low blood glucose levels due to various causes, such as drug treatment, diseases, and hormone or enzyme deficiency. It has been known that secreted glucagon acts on the liver to break down glycogen, thereby inducing the release of glucose and eventually raising blood glucose levels to normal levels. It has also been known that glucagon can lower blood lipid levels by inhibiting fat synthesis in the liver as well promoting fatty acid burning. In addition, glucagon has been known to also act directly or indirectly on white fat to induce fat burning and fat browning, thereby showing an effective weight loss effect (Nat Rev Endocrinol. 11, 329-38 (2015); and Physiol Rev. 97, 721-66 (2017)). This glucagon exhibits activity by acting on glucagon receptor.

Glucagon-like peptide-1 (GLP-1) and glucose-dependent insulinotropic polypeptide (GIP), which are both typical gastrointestinal hormones and neuronal hormones, are known as substances that are involved in the control of blood glucose levels according to food intake.

Recently, there has arisen a need for a substance that can act on glucagon-like peptide-1 (GLP-1) and glucose-dependent insulinotropic polypeptide (GIP) receptors as well as glucagon receptor to increase efficacy or reduce side effects, and therefore, the present inventors have developed peptides capable of acting on glucagon, GLP-1, and GIP receptors and conjugates thereof (WO 2017-116204 and WO 2017-116205).

Blood pressure is an important indicator that needs to be maintained at a certain level in the body, and various drugs are being developed to lower blood pressure when blood pressure levels are temporarily or chronically higher than the normal range. Although there are various causes of temporary or chronic blood pressure elevation, metabolic diseases represented by obesity have an important influence on blood pressure elevation. Actually, a glucagon-like peptide-1 agonist known to be effective in weight loss has recently been reported to have effects of lowering blood pressure and alleviating cardiovascular diseases (N Engl J Med. 375, 311-22 (2016); and N Engl J Med. 375, 1834-44 (2016)). However, the blood pressure lowering effect of the glucagon-like peptide-1 agonist is relatively insignificant since the effect is known to be due to weight loss itself.

Blood vessel dilation is one measure capable of effectively lowering blood pressure, and endothelial nitric oxide synthase (eNOS) has been known to be mainly involved in blood vessel dilation. The endothelial nitric oxide synthase allows the synthesis of nitric oxide (NO), functioning to dilate blood vessels, in vascular endothelial cells, and the synthesized nitric oxide functions as a vasodilator.

Accordingly, efforts are being made to develop effective blood pressure-lowering drugs by discovering substances that have effects of dilating blood vessel and lowering blood pressure by acting directly on blood vessel walls.

### [Disclosure]

### [Technical Problem]

There is a need to develop effective and safe blood pressure-lowering drugs.

### [Technical Solution]

An aspect of the present invention is to provide a pharmaceutical composition for lowering blood pressure, the composition containing a peptide having activities for all of glucagon, GLP-1, and GIP receptors, or a conjugate thereof.

Another aspect of the present invention is to provide a method for lowering blood pressure, the method comprising administering a peptide having activities for all of glucagon, GLP-1, and GIP receptors, or a conjugate thereof, or a composition for lowering blood pressure containing the peptide or the conjugate thereof.

Still another aspect of the present invention is to provide use of a peptide having activities for all of glucagon, GLP-1, and GIP receptors, or a conjugate thereof, for lowering blood pressure.

Still another aspect of the present invention is to provide use of a peptide having activities for all of glucagon, GLP-1, and GIP receptors, or a conjugate thereof, for the preparation of a medicament for use in lowering blood pressure.

### [Advantageous Effects]

The peptide of the present invention having activities for all of glucagon, GLP-1, and GIP receptors shows a blood pressure lowering effect through blood vessel dilation and thus can be used as a drug for a subject with elevated blood pressure.

### [Brief Description of Drawings]

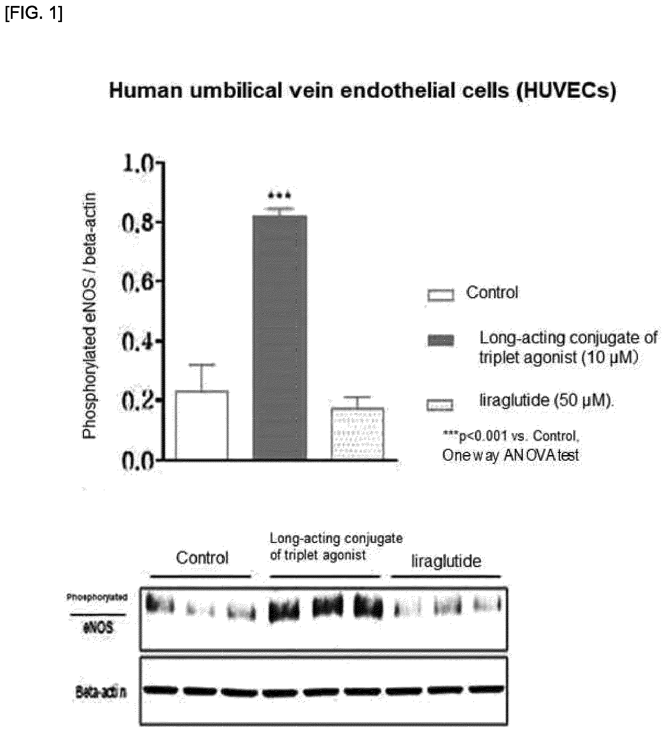

FIG. 1 confirms the blood vessel dilation effect of the long-acting conjugate of SEQ ID NO: 42 by measuring the phosphorylation of endothelial nitric oxide synthase in human umbilical vein endothelial cells.

### [Detailed Description of the Invention]

In accordance with an aspect of the present invention, there is provided a composition containing a peptide having activities for all of glucagon, GLP-1, and GIP receptors, or a conjugate thereof.

In the composition according to an embodiment, the composition is a pharmaceutical composition for lowering blood pressure, which contains a peptide having activities for all of glucagon, GLP-1, and GIP receptors, or a conjugate thereof.

In the composition according to another embodiment, the peptide having activities for all of glucagon, GLP-1, and GIP receptors includes an amino acid sequence of any one of SEQ ID NOS: 1 to 102.

In the composition according to any one of the previous embodiments, the peptide is in the form of a long-acting conjugate, wherein the long-acting conjugate is represented by Formula 1 below:

[Formula 1] X-L-F

wherein X represents a peptide including an amino sequence of any one of SEQ ID NOS: 1 to 102;
L represents a linker containing ethylene glycol repeating units;
F represents an immunoglobulin Fc region; and
"-" represents covalent linkages between X and L and between L and F, respectively.

In the composition according to any one of the previous embodiments, the composition shows a blood pressure lowering effect through blood vessel dilation in a subject.

In the composition according to any one of the previous embodiments, the subject has a metabolic syndrome or liver disease.

In the composition according to any one of the previous embodiments, the peptide is not C-terminally modified or C-terminally amidated.

In the composition according to any one of the previous embodiments, the peptide has a ring formed between amino acid residues.

In the composition according to any one of the previous embodiments, the immunoglobulin Fc region is aglycosylated.

In the composition according to any one of the previous embodiments, the immunoglobulin Fc region is selected from the group consisting of: (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one, or two or more domains, among a CH1 domain, a CH2 domain, a CH3 domain, and a CH4 domain, and an immunoglobulin hinge region or a part of the hinge region; and (f) a dimer of each domain of a heavy chain constant region and a light chain constant region.

In the composition according to any one of the previous embodiments, the immunoglobulin Fc region is a native Fc in which a site capable of forming an inter-disulfide bond is deleted, a native Fc in which some amino acids at the N-terminus are deleted, or a native Fc in which a methionine residue is added at the N-terminus, a complement-binding site is deleted, or an antibody-dependent cell-mediated cytotoxicity (ADCC) site is deleted.

In the composition according to any one of the previous embodiments, the immunoglobulin Fc region is derived from IgG, IgA, IgD, IgE, or IgM.

In the composition according to any one of the previous embodiments, the immunoglobulin Fc region is a hybrid of domains with different origins derived from immunoglobulins selected from the group consisting of IgG, IgA, IgD, IgE, and IgM.

In the composition according to any one of the previous embodiments, the immunoglobulin Fc region has a dimeric form.

In the composition according to any one of the previous embodiments, the immunoglobulin Fc region is a dimer consisting of two polypeptide chains, wherein one end of L is linked to only one of the two polypeptide chains.

In the composition according to any one of the previous embodiments, the immunoglobulin Fc region is an IgG4 Fc region.

In the composition according to any one of the previous embodiments, the immunoglobulin Fc region is an aglycosylated Fc region derived from human IgG4.

In the composition according to any one of the previous embodiments, the immunoglobulin Fc region has a structure in which two polypeptide chains are linked through a disulfide bond, wherein two polypeptide chains are linked through only a nitrogen atom of one of the two chains.

In the composition according to any one of the previous embodiments, the immunoglobulin Fc region includes a monomer of the amino acid sequence of SEQ ID NO: 123.

In the composition according to any one of the previous embodiments, the immunoglobulin Fc region is a homodimer of monomers of the amino acid sequence of SEQ ID NO: 123.

In the composition according to any one of the previous embodiments, the immunoglobulin Fc region is linked through the nitrogen atom of N-terminal proline.

In the composition according to any one of the previous embodiments, F, which is an immunoglobulin Fc region, and X are aglycosylated.

In the composition according to any one of the previous embodiments, the ethylene glycol repeating units are [OCH₂CH₂]ₙ where n is a natural number, wherein n is determined such that the average molecular weight, for example, the number average molecular weight, of a moiety of [OCH₂CH₂]ₙ in the peptide conjugate is 1 kDa to 100 kDa.

In the composition according to any one of the previous embodiments, the value of n is determined such that the average molecular weight, for example, the number average molecular weight, of the moiety of [OCH₂CH₂]ₙ in the peptide conjugate is 10 kDa.

In the composition according to any one of the previous embodiments, in the conjugate, L is linked to F and X by covalent linkages formed by reacting one end of L with an amine group or thiol group of F and reacting the other end of L with an amine group or thiol group of X, respectively.

In the composition according to any one of the previous embodiments, L is polyethylene glycol.

In the composition according to any one of the previous embodiments, the formula weight of a moiety of the ethylene glycol repeating units is in the range of 1 kDa to 100 kDa.

In the composition according to any one of the previous embodiments, the metabolic syndrome is selected from the group consisting of impaired glucose tolerance, hypercholesterolemia, dyslipidemia, obesity, diabetes, hypertension, non-alcoholic steatohepatitis (NASH), atherosclerosis caused by dyslipidemia, atherosclerosis, arteriosclerosis, coronary heart disease, and stroke.

In the composition according to any one of the previous embodiments, the liver disease is selected from the group consisting of alcoholic liver disease, non-alcoholic liver disease, metabolic liver disease, liver fibrosis, liver cirrhosis, fatty liver, hepatitis, viral liver disease, hepatitis, hepatotoxicity, cholestasis, fatty liver, cirrhosis, liver ischemia, liver abscess, hepatic coma, liver atrophy, liver failure, cholestatic liver disease, primary biliary cirrhosis, primary sclerosing cholangitis, and liver cancer.

In the composition according to any one of the previous embodiments, the subject has i) a metabolic disease, ii) a liver disease, or iii) a metabolic disease and a liver disease, accompanied by a hypertensive disease.

In the composition according to any one of the previous embodiments, the subject has non-alcoholic fatty liver disease accompanied by a metabolic disease.

In the composition according to any one of the previous embodiments, the subject has non-alcoholic steatohepatitis (NASH) accompanied by a metabolic disease.

In the composition according to any one of the previous embodiments, the subject has at least one metabolic disease risk factor.

In the composition according to any one of the previous embodiments, the composition has a blood pressure lowering effect in a subject having obesity.

In the composition according to any one of the previous embodiments, the composition has a blood pressure lowering effect in a subject having fatty liver.

In the composition according to any one of the previous embodiments, the composition has a blood pressure lowering effect in a subject having obesity and fatty liver.

In the composition according to any one of the previous embodiments, the composition is used for treating non-alcoholic fatty liver disease in a subject at risk of developing obesity or a metabolic disease.

In the composition according to any one of the previous embodiments, the composition is used for treating non-alcoholic steatohepatitis (NASH) in a subject at risk of developing obesity or a metabolic disease.

In the composition according to any one of the previous embodiments, the peptide or the long-acting conjugate is parenterally administered.

In the composition according to any one of the previous embodiments, the peptide or the long-acting conjugate is subcutaneously administered.

In the composition according to any one of the previous embodiments, the peptide or the long-acting conjugate is parenterally administered once a week.

In the composition according to any one of the previous embodiments, the peptide or the long-acting conjugate is parenterally administered at 0.1 mg to 15 mg once a week.

In the composition according to any one of the previous embodiments, the peptide or the long-acting conjugate is parenterally administered at 1 mg to 10 mg once a week for 4 weeks.

In the composition according to any one of the previous embodiments, the long-acting conjugate is parenterally administered at 1 mg to 10 mg once a week for 4 weeks.

In the composition according to any one of the previous embodiments, the peptide or the long-acting conjugate is parenterally administered at 2 mg, 4 mg, 6 mg, or 8 mg once a week for 4 weeks.

In the composition according to any one of the previous embodiments, the long-acting conjugate is parenterally administered at 2 mg, 4 mg, 6 mg, or 8 mg once a week for 4 weeks.

In accordance with another aspect of the present invention, there is provided a method for lowering blood pressure, the method including administering, to a subject, a peptide having activities for all of glucagon, GLP-1, and GIP receptors, a conjugate thereof, or a composition containing the peptide or the conjugate thereof.

In accordance with still another aspect of the present invention, there is provided use of a peptide having activities for all of glucagon, GLP-1, and GIP receptors, or a conjugate thereof, for lowering blood pressure.

In accordance with still another aspect of the present invention, there is provided use of a peptide having activities for all of glucagon, GLP-1, and GIP receptors, or a conjugate thereof, for the preparation of a medicament for use in lowering blood pressure.

### [Mode for Carrying Out the Invention]

Hereinafter, the present invention will be described in detail.

Each description and embodiment disclosed in this disclosure may also be applied to other descriptions and embodiments. That is, all combinations of various elements disclosed in this disclosure fall within the scope of the present disclosure. Further, the scope of the present invention is not limited by the specific description below.

Throughout the description, not only the typical one-letter and three-letter codes for naturally occurring amino acids, but also three-letter codes generally allowed for other amino acids, such as 2-aminoisobutyric acid (Aib), N-methylglycine (Sar), and α-methyl-glutamic acid, are used. The amino acids mentioned in abbreviations herein are described according to the IUPAC-IUB rules.

| | |
|---|---|
| alanine (Ala, A), | arginine (Arg, R) |
| asparagine (Asn, N), | aspartic acid (Asp, D) |
| cysteine (Cys, C), | glutamic acid (Glu, E) |
| glutamine (Gln, Q), | glycine (Gly, G) |
| histidine (His, H), | isoleucine (Ile, I) |
| leucine (Leu, L), | lysine (Lys, K) |
| methionine (Met, M), | phenylalanine (Phe, F) |
| proline (Pro, P), | serine (Ser, S) |
| threonine (Thr, T), | tryptophan (Trp, W) |
| tyrosine (Tyr, Y), | valine (Val, V) |

Herein, "Aib" may be used interchangeably with "2-aminoisobutyric acid" or "aminoisobutyric acid", and 2-aminoisobutyric acid and aminoisobutyric acid may be used interchangeably with each other.

In accordance with an aspect of the present invention, there is provided a composition containing a peptide having activities for glucagon receptor, glucagon-like peptide-1 (GLP-1) receptor, and glucose-dependent insulinotropic polypeptide (GIP) receptor. Specifically, the composition is a pharmaceutical composition, for lowering blood pressure, containing the peptide or a conjugate thereof.

In an embodiment, the peptide may include an amino acid sequence of any one of SEQ ID NOS: 1 to 102.

In another embodiment, the pharmaceutical composition for lowering blood pressure may be a pharmaceutical composition containing: a pharmaceutically acceptable excipient; and a peptide including an amino acid sequence of any one of SEQ ID NOS: 1 to 102 or a conjugate thereof at a pharmaceutically effective amount.

The composition containing the peptide or the conjugate thereof according to the present invention can lower blood pressure levels by dilating blood vessels in the body.

Specifically, blood vessel dilation and blood pressure lowering by the peptide or the conjugate thereof can be achieved by means of an increase in the activity of endothelial nitric oxide synthase in vascular endothelial cells.

The composition of the present invention can show a blood pressure lowering effect through blood vessel dilation in a subject.

In the present invention, the blood pressure lowering means lowering blood pressure levels. Many blood pressure-lowering drugs are aimed at hypertensive patients, but non-hypertensive patients as well as hypertensive patients may have high blood pressure levels due to other diseases or specific causes, and therefore, blood pressure-lowering drugs that are not limited to patients with specific diseases may have advantages as drugs.

The blood vessel dilation or not can be checked by a reduction in systolic blood pressure (SBP) and diastolic blood pressure (DBP), a change in phosphorylation of endothelial nitric oxide synthase in endothelial cells, and the like.

In the present invention, the term "subject" may refer to a mammal including rats, livestock, and the like, as well as humans in need of lowering blood pressure, and specifically, the subject may have a metabolic syndrome or liver disease, but the subject is not limited to a patient with a specific disease as long as the patient can obtain a beneficial effect through blood pressure lowering. The blood pressure lowering effect by the peptide of the present invention can be shown in not only hypertensive patients but also non-hypertensive patients.

In the present invention, the metabolic syndrome refers to a symptom occurring due to abnormality of at least two of cholesterol, blood pressure, and blood sugar levels, and specifically may mean impaired glucose tolerance, hypercholesterolemia, dyslipidemia, obesity, diabetes, hypertension, non-alcoholic steatohepatitis (NASH), atherosclerosis caused by dyslipidemia, atherosclerosis, arteriosclerosis, coronary heart disease, or stroke, but is not limited thereto.

The liver disease may be alcoholic liver disease, non-alcoholic liver disease, metabolic liver disease, liver fibrosis, liver cirrhosis, fatty liver, hepatitis, viral liver disease, hepatitis, hepatotoxicity, cholestasis, cirrhosis, liver ischemia, liver abscess, hepatic coma, liver atrophy, liver failure, cholestatic liver disease, primary biliary cirrhosis, primary sclerosing cholangitis, or liver cancer, but is not limited thereto.

In many cases, metabolic syndrome or liver disease patients prefer westernized diets and high-salt diets, and such dietary habits may increase the salt concentration in the blood to cause blood pressure elevation. In obesity, dyslipidemia, hypercholesterolemia, atherosclerosis, and others, high blood lipids may disturb blood flow to cause blood pressure elevation. In the liver diseases represented by non-alcoholic steatohepatitis, liver fibrosis, and liver cirrhosis, the dilation of blood vessels present in the liver tissues is inhibited to cause a very high risk of blood pressure elevation. In this regard, the administration of the composition of the present invention having a blood pressure lowering effect through blood pressure dilation can obtain an excellent blood pressure lowering effect.

In an embodiment, a subject to which the composition of the present invention is administered may be a subject having obesity or fatty liver, or a subject having obesity and fatty liver. The administration of the composition of the present invention can obtain a blood pressure lowering effect in a subject having obesity and/or fatty liver, but is not limited thereto.

The composition of the present invention is provided as a blood pressure lowering drug that is not limited to a group of patients with specific diseases since non-hypertensive patients as well as hypertensive patients may have high blood pressure levels due to other diseases or specific causes.

For example, obesity is also a major cause of cardiovascular disease since obesity causes increases in blood lipid and inflammation to narrow blood vessels, resulting in blood pressure elevation.

According to epidemiologic studies, the prevalence of hypertension increases as body mass index increases in both men and women, and body mass index is known to be more closely related to systolic blood pressure than diastolic blood pressure. In the Framingham Heart Study, obesity was predicted to result in a risk of hypertension of 78% in men and 65% in women. The occurrence of hypertension is also associated with abdominal obesity, and thus the risk of hypertension occurring due to abdominal obesity was estimated to correspond to 21-27% in men and 37-57% in women in the USA (NHANES III data). The fact that obesity is an important cause of hypertension can also be seen from several clinical studies targeting patients with essential hypertension showing that a pressure lowering effect was obtained by reducing body weight. Interestingly, obese persons are more prone to hypertension, and hypertensive persons also appear prone to weight gain. The Framingham and Tecumseh studies revealed that future weight gain is significantly higher in hypertensive subjects than in those who are normotensive. Therefore, the relationship between obesity and hypertension is likely to be bidirectional rather than unidirectional. Recently, adipose tissue has been recognized as an endocrine organ and has also been confirmed to produce and secrete several adipokines to have a wide range of effects on systemic metabolism and obesity-causing diseases. In particular, it is known that an increase in leptin associated with diet and energy metabolism is closely related to cardiovascular disease. C-reactive protein (CRP) is also expressed in adipose tissue and involved in the development of leptin resistance. CRP and leptin, along with an increase in inflammatory markers, are closely related to increases in insulin resistance and cardiovascular disease. Obesity also has an adverse influence on hemodynamics and cardiovascular system structures and functions. Obesity increases the total blood volume and cardiac output and increases cardiac workload. Typically, obese patients show higher cardiac output and lower peripheral resistance under the same range of blood pressure conditions. The increase in cardiac output in obesity is mostly due to an increase in stroke volume, and the heart rate also shows a slight increase due to the activation of the sympathetic nervous system. Obese patients are more prone to developing hypertension than normal persons, and weight gain is often associated with blood pressure elevation. Overweight or obese patients frequently undergo left ventricular dilatation due to increased filling pressure and blood volume. Obesity, independently of blood pressure and age, increases left ventricular hypertrophy, and moreover, it causes left atrial enlargement. Left atrial enlargement causes not only an increase in heart failure but also complications such as atrial fibrillation.

In an embodiment, the subject to which the composition of the present invention is administered may be a subject having i) a metabolic disease, ii) a liver disease, or iii) a metabolic disease and a liver disease, accompanied by a hypertensive disease, but any subject that can undergo blood pressure lowering by the composition of the present invention is include without limitation.

In an embodiment, the subject may have non-alcoholic fatty liver disease accompanied by a metabolic disease, but any subject that can undergo blood pressure lowering by the composition of the present invention is include without limitation.

In an embodiment, the subject may have non-alcoholic steatohepatitis (NASH) accompanied by a metabolic disease, but any subject that can undergo blood pressure lowering by the composition of the present invention is include without limitation.

In an embodiment, the subject may have at least one metabolic disease risk factor, but any subject that can undergo blood pressure lowering by the composition of the present invention is include without limitation.

The metabolic disease risk factor may include low HDL cholesterol, high blood pressure, elevated fasting blood sugar, high triglycerides, abdominal obesity, and the like, but is not limited thereto.

Herein, "metabolic disease" may be used interchangeably with "metabolic syndrome".

In an embodiment, the composition may be used for treating non-alcoholic fatty liver disease (NAFLD) in a subject at risk of developing obesity or a metabolic disease, but is not limited thereto.

In an embodiment, the composition may be used for treating non-alcoholic steatohepatitis (NASH) in a subject at risk of developing obesity or a metabolic disease, but is not limited thereto.

The composition of the present invention may contain a peptide having activities for glucagon receptor, GLP-1 receptor, and GIP receptor at a pharmaceutically effective amount, and specifically may contain a peptide (a triple agonist) including, consisting essentially of, or consisting of any one of the amino acid sequences of SEQ ID NOS: 1 to 102, at a pharmaceutically effective amount, but is not limited thereto.

The "peptide having activities for glucagon receptor, GLP-1 receptor, and GIP receptor" of the present invention may be used interchangeably with the terms "triple agonist" or "peptide" herein.

The triple agonist of the present invention may include a peptide including any one of the amino acid sequences of SEQ ID NOS: 1 to 102. Alternatively, a peptide consisting essentially of or consisting of any one of the amino acid sequences of SEQ ID NOS: 1 to 102 may also be included in the triple agonist of the present invention, but is not limited thereto.

Although not particularly limited, the triple agonist having significant levels of activities for glucagon, GLP-1, and GIP receptors may exhibit *in vitro* activities for one or more receptors, specifically two or more receptors, and more specifically all three of the receptors among glucagon, GLP-1, and GIP receptors, of about 0.001% or higher, about 0.01% or higher, about 0.1% or higher, about 1% or higher, about 2% or higher, about 3 % or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, about 100% or higher, about 150% or higher, or about 200% or higher, compared with native ligands for the corresponding receptors (native glucagon, native GLP-1, and native GIP), but any range with a significant increase is included without limitation.

The activities for the receptors may include, for example, those cases where the *in vitro* activities for the receptors are about 0.001% or higher, about 0.01% or higher, about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, about 100% or higher, or about 200% or higher, compared with the native forms. However, the activities for the receptors are not limited thereto.

As used herein, the term "about" refers to a range including ±0.5, ±0.4, ±0.3, ±0.2, ±0.1, and the like, and thus includes all of the values in the range equivalent or similar to those stated after this term, but is not limited thereto.

Reference is made to Experimental Example 1 herein for methods for measuring the *in vitro* activities of such triple agonists, but is not limited thereto.

The peptide retains one or more, two or more, specifically three of the following activities of i) to iii) below, specifically significant activities thereof:
i) activation of GLP-1 receptor; ii) activation of glucagon receptor; and iii) activation of GIP receptor.

In particular, the activation of the receptors may include, for example, those cases where the *in vitro* activities for the receptors are about 0.001 % or higher, about 0.01% or higher, about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, about 100% or higher, about 150% or higher, or about 200% or more, compared with native forms. However, the activation of the receptors is not limited thereto.

The peptide may have an increased *in vivo* half-life compared with any one of native GLP-1, native glucagon, and native GIP, but is not particularly limited thereto.

Although not particularly limited, the peptide may be one which does not occur naturally.

Although described as a peptide "consisting of" a specific SEQ ID NO herein, such description does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide consisting of the amino acid sequence of the corresponding SEQ ID NO, and it would be obvious that even a peptide with such a sequence addition or mutation falls within the scope of the present invention. In other words, if a peptide exhibits at least a certain level of homology and shows activity for glucagon receptor despite having some sequence differences, such a peptide may fall within the scope of the present invention.

For example, reference may be made to WO 2017-116204 and WO 2017-116205 for the peptide of the present invention.

For example, the peptide of the present invention may include a peptide including an amino acid sequence of any one of SEQ ID NOS: 1 to 102, consisting (essentially) of an amino acid sequence of any one of SEQ ID NOS: 1 to 102, or having sequence identity of at least 60%, 65%, 70%, 75%, 80%, 85%, 90%, 91%, 92%, 93%, 94%, or 95% with an amino acid sequence of any one of SEQ ID NOS: 1 to 102, and the peptide of the present invention is not limited to specific sequences as long as the peptide has an effect of dilating blood vessels and lowering blood pressure.

For example, the peptide of the present invention may have an *in vitro* activity, for glucagon receptor playing an important role in the blood pressure lowering mechanism, of about 1% or higher, about 5% or higher, about 10% or higher, about 30% or higher, about 50% or higher, about 100% or higher, or about 200% or higher compared with native glucagon, but is not limited thereto.

Specifically, the peptide of the present invention may also include a peptide including an amino acid sequence of any one of SEQ ID NOS: 21 to 24, 28, 29, 31, 32, 37, 42, 43, 50, 51 to 54, 56, 58, 64 to 73, 75 to 79, 82, 83, 91, and 96 to 102, consisting (essentially) of an amino acid sequence of any one of SEQ ID NOS: 21 to 24, 28, 29, 31, 32, 37, 42, 43, 50, 51 to 54, 56, 58, 64 to 73, 75 to 79, 82, 83, 91, and 96 to 102, or having sequence identity of at least 60%, 65%, 70%, 75%, 80%, 85%, 90%, 91%, 92%, 93%, 94%, or 95% with an amino acid sequence of any one of SEQ ID NOS: 21 to 24, 28, 29, 31, 32, 37, 42, 43, 50, 51 to 54, 56, 58, 64 to 73, 75 to 79, 82, 83, 91, and 96 to 102, and the peptide of the present invention is not limited to specific sequences as long as the peptide has an effect of dilating blood vessels and lowering blood pressure.

As used herein, the term "homology" or "identity" refers to a degree of relatedness between two given amino acid sequences or nucleotide sequences, and the term may be expressed as a percentage.

The terms homology and identity may be often used interchangeably.

The homology, similarity, or identity between any two peptide sequences may be determined by, for example, a known computer algorithm, such as the "FASTA" program, by using default parameters as in Pearson et al (1988) Proc. Natl. Acad. Sci. USA 85:2444. Alternatively, they may be determined using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J. et al., Nucleic Acids Research 12:387 (1984)), BLASTP, BLASTN, FASTA (Atschul, S. F. et al., J Molec Biol 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information Database.

The homology, similarity, or identity of peptides may be determined by comparing sequence information using a GAP computer program (e.g., Needleman et al. (1970), J Mol Biol 48:443) as disclosed in Smith and Waterman, Adv. Appl. Math (1981) 2:482. Briefly, the GAP program defines similarity as the number of aligned symbols (i.e., amino acids) which are similar, divided by the total number of symbols in the shorter of the two sequences. The default parameters for the GAP program may include: (1) a unary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl. Acids Res. 14:6745, as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap open penalty 10, gap extension penalty 0.5); and (3) no penalty for end gaps. Therefore, the term "homology" or "identity" used herein represents the relatedness between sequences.

The peptide of the present invention is characterized by: (i) increasing the phosphorylation of endothelial nitric oxide synthase (eNOS); and/or (ii) increasing the expression and activity of endothelial nitric oxide synthase. The endothelial nitric oxide synthase, which is an enzyme that synthesizes nitric oxide (NO) functioning to dilate blood vessels, from L-arginine in vascular endothelial cells, is known as an important regulator for blood flow and blood pressure regulation. It is known that the suppression of nitric oxide production due to the inhibition of endothelial nitric oxide synthase causes endothelial dysfunction in which blood vessel relaxation is difficult. The peptide or the composition containing the peptide of the present invention can dilate blood vessels of a subject by enhancing the activity of endothelial nitric oxide synthase. The peptide of the present invention can show a blood pressure lowering effect of reducing systolic blood pressure (SBP) and diastolic blood pressure (DBP), through the blood vessel dilation.

Such a peptide may include an intramolecular bridge (e.g., a covalent bridge or non-covalent bridge), and specifically may be in the form of including a ring, for example, may be in the form of including a ring formed between the amino acid at position 16 and the amino acid at position 20 of the peptide, but the peptide is not particularly limited thereto.

Non-limiting examples of the ring may include a lactam bridge (or a lactam ring).

Additionally, the peptide includes all of those which are modified to include a ring, or to include amino acids capable of forming a ring at a target position.

For example, the pair of the amino acids at positions 16 and 20 in the peptide may be substituted with glutamic acid or lysine, which can form a ring, but the present invention is not limited thereto.

Such a ring may be formed between amino acid side chains in the peptide, for example, in the form of a lactam ring formed between a side chain of lysine and a side chain of glutamic acid, but is not particularly limited thereto.

Examples of the peptide prepared by a combination of these methods include a peptide, of which the amino acid sequence differs from that of native glucagon in at least one amino acid, from which the α-carbon of the amino acid residue at the N-terminus is removed, and retains activities for glucagon receptor, GLP-1 receptor, and GIP receptor, but are not limited thereto. Peptides applicable to the present invention can be prepared by combining various methods for the preparation of analogs.

Additionally, in the peptide of the present invention, some amino acids may be substituted with other amino acids or non-natural compounds to avoid the recognition by an agonist protease, for increasing the *in vivo* half-life of the peptide, but the peptide is not particularly limited thereto.

Specifically, the peptide of the present invention may be a peptide in which the *in vivo* half-life is increased by avoiding the recognition by a degradation enzyme through a substitution of the second amino acid in the amino acid sequence of the peptide, but any substitution or change of amino acids to avoid recognition by an *in vivo* degradation enzyme is included without limitation.

Such a modification for peptide preparation includes all of: modifications using L-type or D-type amino acids and/or non-native amino acids; and/or modifications of native sequence, for example, a variation of a side chain functional group, an intramolecular covalent linkage (*e*.*g*., ring formation between side chains), methylation, acylation, ubiquitination, phosphorylation, aminohexanation, biotinylation, or the like.

In addition, such a variation also includes all the addition of one or more amino acids to the amino and/or carboxy terminus of native glucagon.

The substituted or added amino acids may be not only the 20 amino acids that are commonly found in human proteins, but also atypical amino acids or those which do not occur naturally. Commercial sources of atypical amino acids may include Sigma-Aldrich, ChemPep, and Genzyme Pharmaceuticals. The peptides including these amino acids and typical peptide sequences can be synthesized and purchased from commercial peptide suppliers, for example, American Peptide Company and Bachem in the USA, or Antigen in Korea.

Amino acid derivatives may also be accessible in a similar manner, and for example, 4-imidazoacetic acid or the like may be used.

The peptide according to the present invention may be in a varied form in which the N-terminus and/or C-terminus is chemically modified or protected by organic groups, or amino acids are added to the terminus of the peptide, for its protection from proteases *in vivo* while increasing its stability.

In particular, a chemically-synthesized peptide has electrically charged Nand C-termini, and thus for elimination of these charges, the N-terminus may be acetylated and/or the C-terminus may be amidated, but the peptide is not particularly limited thereto.

Specifically, the N-terminus or the C-terminus of the peptide of the present invention may have an amine group (-NH₂) or a carboxy group (-COOH), but is not limited thereto.

The peptide according to the present invention may include a peptide, of which the C-terminus is amidated or has a free carboxy (-COOH) group, or a peptide, of which the C-terminus is not modified, but is not limited thereto.

In an embodiment, the peptide may be C-terminally amidated, but is not limited thereto.

In an embodiment, the peptide may be aglycosylated, but is not limited thereto.

The peptide of the present invention may be synthesized through solid-phase synthesis, produced by a recombinant method, or prepared by commercial request, but is not limited thereto.

In addition, the peptide of the present invention may be synthesized depending on the length thereof by a method well known in the art, for example, an automatic peptide synthesizer, and may be produced by genetic engineering technology.

Specifically, the peptide of the present invention may be prepared by a standard synthesis method, a recombinant expression system, or any other method known in the art. Therefore, the peptide according to the present invention may be synthesized by a number of methods including, for example, the following:
(a) a method of synthesizing a peptide by means of solid phase or liquid phase methodology either stepwise or by fragment assembling, followed by isolation and purification of the final peptide product;
(b) a method of expressing a nucleic acid construct encoding a peptide in a host cell and recovering the expression product from the host cell culture;
(c) a method of performing an *in vitro* cell-free expression of a nucleic acid construct encoding a peptide and recovering the expression product therefrom; or
a method of obtaining peptide fragments by any combination of the methods (a), (b), and (c), obtaining the peptide by linking the fragments, and then recovering the peptide.

In addition, the peptide having activities for glucagon receptor, GLP-1 receptor, and GIP receptor may be in the form of a long-acting conjugate in which a biocompatible material for increasing the *in vivo* half-life of a peptide having activities for glucagon receptor, GLP-1 receptor, and GIP receptor is conjugated to the peptide. The composition of the present invention may include the long-acting conjugate. Herein, the biocompatible material may be used interchangeably with a carrier.

In the present invention, a conjugate of the peptide can exhibit an increase in the duration of efficacy compared with the peptide to which a carrier is not conjugated, and in the present invention, such a conjugate is referred to as a "long-acting conjugate".

As used herein, the term "long-acting conjugate" or "conjugate" has a structure in which a biocompatible material is conjugated to the peptide, and such a conjugate can exhibit an increase in the duration of efficacy compared with the peptide to which the biocompatible material is not conjugated. In the long-acting conjugate, the biocompatible material may be linked to the peptide by a covalent linkage, but is not particularly limited thereto. In the present invention, the peptide, which is one element of the conjugate, may be a peptide having activities for glucagon receptor, GLP-1 receptor, and GIP receptor, and specifically a peptide or fragment including an amino acid sequence of any one of the amino acid sequences of SEQ ID NO: 1 to 102, and the biocompatible material is a substance capable of increasing the half-life of the peptide and corresponds to one element of a moiety constituting the conjugate.

As used herein, the term "biocompatible material" refers to a substance that can be conjugated to a physiologically active substance (the peptide of the present invention) to thereby increase the duration of efficacy of the physiologically active substance compared with a physiologically active substance to which a biocompatible material moiety or carrier is not conjugated. The biocompatible material may be covalently linked to a physiologically active substance, but is not particularly limited thereto.

The biocompatible material may be selected from the group consisting of a polymer, a fatty acid, cholesterol, albumin and a fragment thereof, an albuminbinding substance, a polymer of repeating units of particular amino acid sequences, an antibody, an antibody fragment, an FcRn-binding substance, an *in vivo* connective tissue, a nucleotide, fibronectin, transferrin, a saccharide, heparin, and elastin, but is not limited thereto.

For example, the FcRn-binding substance may be an immunoglobulin Fc region, and more specifically an IgG Fc region, but is not particularly limited thereto.

At least one amino acid side chain within the peptide of the present invention may be joined to such a biocompatible material to increase solubility and/or half-life *in vivo,* and/or increase bio-availability thereof. Such a modification can also reduce the clearance of therapeutic proteins and peptides.

The biocompatible materials described above may be water-soluble (amphipathic or hydrophilic) and/or non-toxic and/or pharmaceutically acceptable.

These conjugates may be those which do not occur naturally.

In a specific embodiment of the present invention, the long-acting conjugate refers to a form in which an immunoglobulin Fc region is linked to a peptide having activities for glucagon receptor, GLP-1 receptor, and GIP receptor. Specifically, the conjugate may be one in which an immunoglobulin Fc region is covalently linked to a peptide having activities for glucagon receptor, GLP-1 receptor, and GIP receptor via a linker, but is not particularly limited thereto.

In an embodiment of the present invention, the long-acting conjugate may be represented by Formula 1 below, but is not limited thereto:

[Formula 1] X-L-F

wherein X represents a peptide including an amino sequence of any one of SEQ ID NOS: 1 to 102;
L represents a linker containing ethylene glycol repeating units;
F represents an immunoglobulin Fc region; and
"-" represents covalent linkages between X and L and between L and F, respectively.

In the long-acting conjugate of Formula 1, X may be the above-described peptide (triple agonist) having activities for glucagon receptor, GLP-1 receptor, and GIP receptor, and specifically may be a peptide including an amino acid sequence of any one of SEQ ID NOS: 1 to 102, or a peptide consisting essentially of or consisting of an amino acid sequence of any one of SEQ ID NOS: 1 to 102, but is not limited thereto.

Herein, the term "long-acting conjugate of Formula 1" refers to a form in which a peptide including an amino acid sequence of any one of SEQ ID NOS: 1 to 102 is linked to an immunoglobulin Fc region via a linker, and the conjugate can exhibit an increase in the duration of efficacy compared with a peptide including an amino acid sequence of any one of the amino acid sequences of SEQ ID NOS: 1 to 102, to which the immunoglobulin Fc region is not conjugated.

The conjugate of the present invention, even in the form of a conjugate, can exhibit significant activities for glucagon receptor, GLP-1 receptor, and GIP receptor, and thus can also exert a blood pressure lowering effect through blood vessel dilation.

Specifically, the conjugate of the present invention can exhibit *in vitro* activities for glucagon receptor, GLP-1 receptor, and/or GIP receptor, of about 0.01 % or higher, about 0.1% or higher, about 0.2% or higher, about 0.5% or higher, about 0.7% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, or about 100% or higher, compared with native forms, but is not limited thereto.

For the purposes of the present invention, the peptide or the conjugate thereof can exhibit activities for glucagon receptor, GLP-1 receptor, and/or GIP receptor, of about 0.01% or higher, about 0.1% or higher, about 1% or higher, about 2% or higher, about 3% or higher, about 4% or higher, about 5% or higher, about 10% or higher, about 20% or higher, about 30% or higher, about 40% or higher, about 50% or higher, about 60% or higher, about 70% or higher, about 80% or higher, about 90% or higher, or about 100% or higher, compared with native forms, but is not limited thereto.

The composition of the present invention may contain (i) a peptide having activities for glucagon receptor, GLP-1 receptor, and GIP receptor, or (ii) a long-acting conjugate of the peptide having activities for glucagon receptor, GLP-1 receptor, and GIP receptor, and the long-acting conjugate can show an excellent blood pressure lowering effect through an increase in the *in vivo* duration of efficacy.

In the long-acting conjugate, F is a substance capable of increasing the half-life of X, that is, a peptide including an amino acid sequence of any one of SEQ ID NOS: 1 to 102, and corresponds to one element of a moiety constituting the conjugate of the present invention.

In the long-acting conjugate of Formula 1, the linkage between X, which is a peptide including an amino acid sequence of any one of SEQ ID NOS: 1 to 102, and an immunoglobulin Fc region may be a physical or chemical linkage, or a non-covalent or covalent linkage, and specifically may be a covalent linkage, but is not limited thereto.

X may be linked to F via a linker (L). More specifically, X and L and L and F may be linked to each other through a covalent linkage, wherein in the conjugate, X, L, and F are linked to each other through a covalent linkage in the order shown in Formula 1.

Specifically, the method for linking X, which is a peptide including an amino acid sequence of any one of SEQ ID NOS: 1 to 102, and an immunoglobulin Fc region in the long-acting conjugate of Formula 1 is not particularly limited, but the peptide including an amino acid sequence of any one of SEQ ID NOS: 1 to 102 and the immunoglobulin Fc region may be linked to each other via a linker.

F may be an immunoglobulin Fc region and, more specifically, the immunoglobulin Fc region may be derived from IgG, but is not particularly limited thereto.

In the present invention, the term "immunoglobulin Fc region" refers to a region that includes heavy chain constant region 2 (CH2) and/or heavy chain constant region 3 (CH3), excluding heavy chain and light chain variable regions in an immunoglobulin. The immunoglobulin Fc region may be one element constituting a moiety of the conjugate of the present invention.

In the present invention, an Fc region includes not only the native sequence obtained by papain digestion of immunoglobulins, but also derivatives thereof, for example, variants in which one or more amino acid residues in the native sequence are modified by deletion, insertion, non-conservative or conservative substitution, or a combination thereof and is thus different from that of the native form. The derivatives, substituent, and variants are required to retain FcRn-binding ability. In the present invention, F may be a human immunoglobulin region, but is not limited thereto. F has a structure in which two polypeptide chains are linked by a disulfide bond, wherein the two polypeptide chains are linked through only a nitrogen atom of one of the two chains. The linking through a nitrogen atom may be linking to the epsilon amino atoms of lysine or the N-terminus amino group through reductive amination.

The term "reductive amination" refers to a reaction in which an amine group or an amino group of one reactant reacts with an aldehyde (i.e., a functional group capable of reductive amination) of another reactant to form an amine, and then an amine linkage is formed by reduction, and reductive amination is an organic synthetic reaction widely known in the art.

In an embodiment, F may be linked through a nitrogen atom of the N-terminus proline thereof, but is not limited thereto.

The immunoglobulin Fc region may be one element constituting a moiety of the conjugate of Formula 1 of the present invention, and may correspond to F in Formula 1.

This immunoglobulin Fc region may include a hinge region in a heavy chain constant region, but is not limited thereto.

In the present invention, the immunoglobulin Fc region may include a specific hinge sequence at the N-terminus.

As used herein, the term "hinge sequence" refers to a site which is located on a heavy chain and forms a dimer of the immunoglobulin Fc region through an inter-disulfide bond.

In the present invention, the hinge sequence may be mutated to have only one cysteine residue by deletion of a part in the hinge sequence having the following amino acid sequence, but is not limited thereto:
Glu-Ser-Lys-Tyr-Gly-Pro-Pro-Cys-Pro-Ser-Cys-Pro (SEQ ID NO: 103).

The hinge sequence may include only one cysteine residue by deletion of the 8th or 11th residues (cysteine) in the hinge sequence of SEQ ID NO: 103. The hinge sequence of the present invention may be composed of 3 to 12 amino acids including only one cysteine residue, but is not limited thereto. More specifically, the hinge sequence of the present invention may have the following sequences:
Glu-Ser-Lys-Tyr-Gly-Pro-Pro-Pro-Ser-Cys-Pro (SEQ ID NO: 104),
Glu-Ser-Lys-Tyr-Gly-Pro-Pro-Cys-Pro-Ser-Pro (SEQ ID NO: 105),
Glu-Ser-Lys-Tyr-Gly-Pro-Pro-Cys-Pro-Ser (SEQ ID NO: 106),
Glu-Ser-Lys-Tyr-Gly-Pro-Pro-Cys-Pro-Pro (SEQ ID NO: 107),
Lys-Tyr-Gly-Pro-Pro-Cys-Pro-Ser (SEQ ID NO: 108),
Glu-Ser-Lys-Tyr-Gly-Pro-Pro-Cys (SEQ ID NO: 109),
Glu-Lys-Tyr-Gly-Pro-Pro-Cys (SEQ ID NO: 110),
Glu-Ser-Pro-Ser-Cys-Pro (SEQ ID NO: 111),
Glu-Pro-Ser-Cys-Pro (SEQ ID NO: 112),
Pro-Ser-Cys-Pro (SEQ ID NO: 113),
Glu-Ser-Lys-Tyr-Gly-Pro-Pro-Ser-Cys-Pro (SEQ ID NO: 114),
Lys-Tyr-Gly-Pro-Pro-Pro-Ser-Cys-Pro (SEQ ID NO: 115),
Glu-Ser-Lys-Tyr-Gly-Pro-Ser-Cys-Pro (SEQ ID NO: 116),
Glu-Ser-Lys-Tyr-Gly-Pro-Pro-Cys (SEQ ID NO: 117),
Lys-Tyr-Gly-Pro-Pro-Cys-Pro (SEQ ID NO: 118),
Glu-Ser-Lys-Pro-Ser-Cys-Pro (SEQ ID NO: 119),
Glu-Ser-Pro-Ser-Cys-Pro (SEQ ID NO: 120),
Glu-Pro-Ser-Cys (SEQ ID NO: 121), and Ser-Cys-Pro (SEQ ID NO: 122).

More specifically, the hinge sequence may include an amino acid sequence of SEQ ID NO: 113 (Pro-Ser-Cys-Pro) or SEQ ID NO: 122 (Ser-Cys-Pro), but is not limited thereto.

The immunoglobulin Fc region of the present invention may be in the form in which two molecules of the immunoglobulin Fc chain form a dimer due to the presence of a hinge sequence, and the conjugate of Formula 1 of the present invention may be in the form in which one end of the linker is linked to one chain of the dimeric immunoglobulin Fc region, but is not limited thereto.

As used herein, the term "N-terminus" refers to an amino terminus of a protein or polypeptide, and may include the outermost end of the amino terminus, or 1, 2, 3, 4, 5, 6, 7, 8, 9, or 10 or more amino acids from the outermost end. In the immunoglobulin Fc region of the present invention, a hinge sequence may be included in the N-terminus thereof, but is not limited thereto.

The immunoglobulin Fc region of the present invention may be an extended Fc region including a part or the entirety of heavy chain constant region 1 (CH1) and/or light chain constant region 1 (CL1), excluding only heavy chain and light chain variable regions of an immunoglobulin, as long as the immunoglobulin Fc region has a substantially equivalent or improved effect compared with the native form. Alternatively, the immunoglobulin Fc region of the present invention may be a region having a deletion of a considerably long partial amino acid sequence corresponding to CH2 and/or CH3.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one, or two or more domains among a CH1 domain, a CH2 domain, a CH3 domain, and a CH4 domain and an immunoglobulin hinge region (or a part of the hinge region); and 6) a dimer of each domain of a heavy chain constant region and a light chain constant region, but is not limited thereto. However, the immunoglobulin Fc region of the present invention is not limited thereto.

In the present invention, the immunoglobulin Fc region may be a dimer or multimer consisting of single-chain immunoglobulins consisting of domains of the same origin, but is not limited thereto.

In an embodiment of the long-acting conjugate of the present invention, the immunoglobulin Fc region F is a dimer consisting of two polypeptide chains, wherein the dimeric Fc region F and X may be covalently linked to each other via one identical linker L containing ethylene glycol repeating units. In a specific embodiment, X is covalently linked to only one polypeptide chain of the two polypeptide chains of the dimeric Fc region F via the linker L. In a more specific embodiment, only one X molecule is covalently linked via L to one polypeptide chain, to which X is linked, of the two polypeptide chains of the dimeric Fc region F. In a most specific embodiment, F is a homodimer.

In another embodiment, the immunoglobulin Fc region F is a dimer consisting of two polypeptide chains, and one end of L is linked to only one polypeptide chain of the two polypeptide chains, but is not limited thereto.

In another embodiment of the long-acting conjugate of the present invention, two molecules of X may be symmetrically linked to one Fc region in a dimeric form. The immunoglobulin Fc and X may be linked to each other via a non-peptide linker. However, the present invention is not limited to the above-described embodiments.

The immunoglobulin Fc region of the present invention includes not only the native amino acid sequence as well as a sequence derivative thereof. The amino acid sequence derivative refers to an amino acid sequence which is different from the native amino acid sequence by a deletion, an insertion, or a non-conservative or conservative substitution of at least one amino acid residue, or a combination thereof.

For example, the amino acid residues at positions 214 to 238, 297 to 299, 318 to 322, or 327 to 331, which are known to be important for linkage in IgG Fc, may be used as appropriate sites for variation.

In addition, various types of derivatives are possible, for example, by removing a region capable of forming an inter-disulfide bond, deleting some amino acid residues at the N-terminus of native Fc, or adding a methionine residue at the N-terminus of native Fc. In addition, in order to remove effector functions, a complement-binding site, for example, a C1q-binding site, may be removed, and an antibody-dependent cell-mediated cytotoxicity (ADCC) site may be removed. Techniques for preparing such sequence derivatives of the immunoglobulin Fc region are disclosed in WO 97/34631 and WO 96/32478, and the like.

Amino acid exchanges in a protein or peptide that do not alter the entire activity of a molecule are well known in the art (H. Neurath, R. L. Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, or the like.

The above-described Fc derivatives exhibit a biological activity equivalent to that of the Fc region of the present invention, and may be obtained by improving the structural stability of the Fc region against heat, pH, and the like.

Such an Fc region may be obtained from a native form isolated from living bodies of humans or animals, such as cows, goats, pigs, mice, rabbits, hamsters, rats, and guinea pigs, or may be a recombinant form obtained from transformed animal cells or microorganisms, or derivatives thereof. In particular, a method of obtaining from a native form is a method in which the whole immunoglobulin is isolated from a living body of a human or animal and then treated with a protease. The native form may be digested into Fab and Fc when treated with papain and digested into pF'c and F(ab)₂ when treated with pepsin. These may be separated into Fc or pF'c by size-exclusion chromatography or the like. In a more specific embodiment, the immunoglobulin Fc region is a recombinant immunoglobulin Fc region obtained from a microorganism.

In addition, the immunoglobulin Fc region may have native glycans, increased or decreased glycans compared with the native form, or be in a deglycosylated form. The increase, decrease, or removal of the immunoglobulin Fc glycans may be attained by using conventional methods, such as a chemical method, an enzymatic method, and a genetic engineering method using a microorganism. The immunoglobulin Fc region obtained by removal of glycans from Fc shows a significant deterioration in binding affinity to the complement C1q and a decrease or elimination in antibody-dependent cytotoxicity or complement-dependent cytotoxicity, and thus cause no unnecessary immune response *in vivo.* In this regard, a deglycosylated or aglycosylated immunoglobulin Fc region may be a more suitable form to meet the original purpose of the present invention as a drug carrier.

As used herein, the term "deglycosylation" refers to enzymatic elimination of sugars from an Fc region, and the term "aglycosylation" means an aglycosylated Fc region produced in prokaryotes, more specifically *E*. *coli.*

Meanwhile, the immunoglobulin Fc region may be originated from humans, or other animals including cows, goats, pigs, mice, rabbits, hamsters, rats, and guinea pigs, and in a more specific embodiment, the immunoglobulin Fc region is originated from humans.

In addition, the immunoglobulin Fc region may be an Fc region derived from IgG, IgA, IgD, IgE, IgM, or a combination or hybrid thereof. In a still more specific embodiment, the immunoglobulin Fc region is derived from IgG or IgM, which is most abundant in the human blood, and in a still more specific embodiment, the immunoglobulin Fc region is derived from IgG, which is known to increase the halflives of ligand-binding proteins. In a still more specific embodiment, the immunoglobulin Fc region is an IgG4 Fc region, and in a most specific embodiment, the immunoglobulin Fc region is an aglycosylated Fc region derived from human IgG4, but is not limited thereto.

In a specific embodiment, the immunoglobulin Fc fragment is a region of human IgG4 Fc, may be in the form of a homodimer in which two monomers are linked through a disulfide bond (inter-chain form) between cysteines, which are the third amino acid of each monomer. In particular, each monomer of the homodimer independently have/may have a disulfide bond between cysteines at positions 35 and 95 and a disulfide bond between cysteines at positions 141 and 199, that is, two disulfide bonds (intra-chain form). With respect to the number of amino acids, each monomer may consist of 221 amino acids, and the number of the amino acids forming the homodimer may be a total of 442, but the number of amino acids is not limited thereto. Specifically, in the immunoglobulin Fc fragment, two monomers having the amino acid sequence of SEQ ID NO: 123 (consisting of 221 amino acids) form a homodimer through a disulfide bond between cysteines, which are the amino acid at position 3 of each monomer, wherein the monomers of the homodimer independently form an intra-disulfide bond between the cysteines at positions 35 and 95 and an intra-disulfide bond between the cysteines at positions 141 and 199, respectively, but the immunoglobulin Fc fragment is not limited thereto.

F in Formula 1 may include a monomer having the amino acid sequence of SEQ ID NO: 123, wherein F may be a homodimer of monomers having the amino acid sequence of SEQ ID NO: 123, but is not limited thereto.

In an embodiment, the immunoglobulin Fc region may be a homodimer including the amino acid sequence of SEQ ID NO: 124 (consisting of 442 amino acids), but is not limited thereto.

In an embodiment, the immunoglobulin Fc region and X may not be glycosylated, but are not limited thereto.

As used herein, the term "combination" means that when a dimer or multimer is formed, a polypeptide encoding the single-chain immunoglobulin Fc region of the same origin forms a bond with a single-chain polypeptide of a different origin. That is, a dimer or multimer can be prepared from two or more regions selected from the group consisting of IgG Fc, IgA Fc, IgM Fc, IgD Fc, and IgE Fc regions.

As used herein, the term "hybrid" means that a sequence corresponding to two or more immunoglobulin Fc regions of different origins is present in a single-chain immunoglobulin constant region. In the present invention, several types of hybrid are possible. That is, domains composed of 1 to 4 domains selected from the group consisting of CH1, CH2, CH3, and CH4 of IgG Fc, IgM Fc, IgA Fc, IgE Fc, and IgD Fc can be hybridized, and may include a hinge region.

Meanwhile, IgG may also be divided into IgG1, IgG2, IgG3, and IgG4 subclasses, and a combination or hybridization thereof can also be made in the present invention. Specifically, IgG may be IgG2 and IgG4 subclasses, and more specifically an Fc fragment of IgG4 having little effector function such as complement dependent cytotoxicity (CDC).

The above-described conjugate may have an increase in the duration of efficacy compared with native GLP-1, GIP, or glucagon or with X which is not modified with F, and such a conjugate is not only in the above-described form, but also in the form encapsulated in biodegradable nanoparticles, but is not limited thereto.

Meanwhile, in Formula 1 above, L is a non-peptide linker, for example, may be a linker containing ethylene glycol repeating units.

In the present invention, the "non-peptide linker" includes a biocompatible polymer having two or more repeating units linked to each other. The repeating units are linked to each other by any covalent linkage but not a peptide linkage. The non-peptide linker may be one element constituting a moiety of the conjugate of the present invention, and corresponds to L in Formula 1.

As the non-peptide linker usable in the present invention, any polymer that has resistance to *in vivo* protease may be used without limitation. In the present invention, the non-peptide linker may be used interchangeably with a non-peptide polymer.

In the present invention, the peptide linker includes reactive groups at the ends thereof and thus may form a conjugate by reaction with the other elements constituting the conjugate. When a non-peptide linker having reactive functional groups at both ends thereof binds to X and F in Formula 1 through the respective reactive groups to form a conjugate, the non-peptide linker or non-peptide polymer may be named a non-peptide polymer linker moiety or a non-peptide linker moiety.

Although not particularly limited, the non-peptide linker may be a linker containing ethylene glycol repeating units, and for example, may be polyethylene glycol, and derivatives thereof already known in the art and derivatives that can be easily prepared within the level of skill in the art also fall within the scope of the present invention.

The repeating units of the non-peptide linker may be ethylene glycol repeating units, and specifically, the non-peptide linker may include, at ends thereof, functional groups for use in the preparation of a conjugate before being configured into the conjugate, while including ethylene glycol repeating units. The long-acting conjugate according to the present invention may be in the form in which X and F are linked through the functional groups, but the long-acting conjugate is not limited thereto. In the present invention, the non-peptide linker may include two, or three or more functional groups, and each functional group may be the same as or different from each other, but is not limited thereto.

Specifically, the linker may be polyethylene glycol (PEG) represented by Formula 2 below, but is not limited thereto: 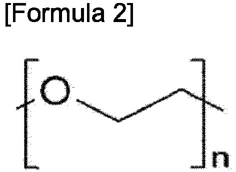 wherein n is 10 to 2,400, n is 10 to 480, or n is 50 to 250, but is not limited thereto.

In the long-acting conjugate, the PEG moiety may include not only the - (CH₂CH₂O)ₙ- structure, but also an oxygen atom interposed between a linking element and the -(CH₂CH₂O)ₙ- structure, but the PEG moiety is not limited thereto.

In an embodiment, the ethylene glycol repeating unit may be represented by, for example, [OCH₂CH₂]ₙ, wherein the value of n is a natural number, and the average molecular weight, for example, the number average molecular weight of a moiety of [OCH₂CH₂]ₙ in the peptide conjugate may be set to more than 0 kDa and less than or equal to about 100 kDa, but is not limited thereto. As another example, the value of n is a natural number, and the average molecular weight, for example, the number average molecular weight of a moiety of [OCH₂CH₂]ₙ in the peptide conjugate may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, or about 10 kDa, but is not limited thereto.

In a specific embodiment, the conjugate may have a structure in which the peptide (X) and the immunoglobulin Fc region (F) are covalently linked via a linker containing ethylene glycol repeating units, but is not limited thereto.

The polyethylene glycol is a term encompassing all of the forms of homopolymers of ethylene glycol, PEG copolymers, and monomethyl-substituted PEG polymers (mPEG), but is not particularly limited thereto.

As the non-peptide linker usable in the present invention, any polymer that has resistance to proteases *in vivo* and contains ethylene glycol units may be used without limitation. The molecular weight of the non-peptide polymer may be in the range of more than 0 kDa and less than or equal to about 100 kDa, or about 1 kDa to about 100 kDa, and specifically about 1 kDa to about 20 kDa, or about 1 kDa to about 10 kDa, but is not limited thereto. In addition, the non-peptide linker of the present invention, which is linked to the polypeptide corresponding to F, may include not only a single type of polymer but also a combination of different types of polymers.

In a specific embodiment, both ends of the linker may be linked to a thiol group, an amino group, or a hydroxyl group of the immunoglobulin Fc region and a thiol group, an amino group, an azide group, or a hydroxyl group of the peptide (X), respectively, but are not limited thereto.

Specifically, the linker may include, at both ends thereof, reactive groups capable of binding to the immunoglobulin Fc region and the peptide (X), respectively, specifically reactive groups capable of binding to a thiol group of cysteine; an amino group located at the N-terminus, lysine, arginine, glutamine, and/or histidine; and/or a hydroxyl group located at the C-terminus in the immunoglobulin Fc region; and a thiol group of cysteine; an amino group of lysine, arginine, glutamine, and/or histidine; an azide group of azido-lysine; and/or a hydroxyl group in the peptide (X), but is not limited thereto.

More specifically, each of the reactive groups of the linker may be at least one selected from the group consisting of an aldehyde group, a maleimide group, and a succinimide derivative, but is not limited thereto.

In the above, examples of the aldehyde group may include a propionaldehyde group or a butyraldehyde group, but are not limited thereto.

In the above, examples of the succinimide derivative may include succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, and succinimidyl carbonate, but are not limited thereto.

The linker may be linked to the immunoglobulin Fc region F and the peptide (triple agonist) X via such reactive groups to be converted into a linker moiety.

In addition, a final product produced by reductive amination through aldehyde linkage is still more stable than one obtained through amide linkage. The aldehyde reactive group selectively reacts with the N-terminus at low pH while forming a covalent linkage with a lysine residue at high pH, for example, at pH 9.0.

In addition, the reactive groups of both ends of the non-peptide linker may be the same as or different from each other, and for example, aldehyde groups may be provided at both ends, or a maleimide group may be provided at one end and an aldehyde group, a propionaldehyde group, or a butyraldehyde group may be provided at the other end. However, the reactive groups are not particularly limited thereto as long as F, specifically an immunoglobulin Fc region, and X can be linked to both ends of the non-peptide linker.

For example, the non-peptide linker may have a maleimide group as a reactive group at one end and an aldehyde group, a propionaldehyde group, a butyraldehyde group, or the like at the other end.

When polyethylene glycol having hydroxyl reactive groups at both ends is used as a non-peptide polymer, the long-acting protein conjugate of the present invention may be prepared by activating the hydroxyl groups into various reactive groups through known chemical reactions or by using commercially available polyethylene glycol having modified reactive groups.

In a specific embodiment, the non-peptide polymer may be linked to a cysteine residue of X, more specifically a -SH group of cysteine, but is not limited thereto.

For example, the non-peptide polymer may be linked to the cysteine residue at position 10, the cysteine residue at position 13, the cysteine residue at position 15, the cysteine residue at position 17, the cysteine residue at position 19, the cysteine residue at position 21, the cysteine residue at position 24, the cysteine residue at position 28, the cysteine residue at position 29, the cysteine residue at position 30, the cysteine residue at position 31, the cysteine residue at position 40, or the cysteine residue at position 41 in the peptide corresponding to X, but is not particularly limited.

Specifically, a reactive group of the non-peptide polymer may be linked to the -SH group of the cysteine residue, and all of those described above are applied to the reactive group. When maleimide-PEG-aldehyde is used, the maleimide group may be linked to the -SH group of X through a thioether linkage, and the aldehyde group may be linked to the -NH₂ group of F, specifically the immunoglobulin Fc region through reductive amination, but is not limited thereto, and this corresponds to one example.

In another specific embodiment, the non-peptide polymer may be linked to a lysine residue of X, more specifically the amino group of the lysine, but is not limited thereto.

In the conjugate, the reactive group of the non-peptide linker may be linked to -NH₂ located at the N-terminus of the immunoglobulin Fc region, and this corresponds to one example.

When maleimide-PEG-aldehyde is used, the maleimide group may be linked to the -SH group of the peptide through a thioether linkage, and the aldehyde group may be linked to the -NH₂ group of the immunoglobulin Fc region through reductive alkylation, but is not limited thereto.

Through such reductive alkylation, an amino group at the N-terminus of the immunoglobulin Fc region is linked to an oxygen atom located at one end of PEG via a linker reactive group having a structure of -CH₂CH₂CH₂- to form a structure, like - PEG-O-CH₂CH₂CH₂NH-immunoglobulin Fc, and through a thioether linkage, a structure in which one end of PEG is linked to a sulfur atom located at cysteine of the peptide may be formed. The above-described thioether linkage may contain the structure of

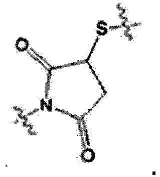

However, the non-peptide polymer is not particularly limited to the above embodiment, and this corresponds to one example.

In the conjugate, the reactive group of the linker may be linked to -NH₂ located at the N-terminus of the immunoglobulin Fc region, but this corresponds to one example.

In the conjugate, the peptide according to the present invention may be linked to a linker having a reactive group through the C-terminus thereof, but this corresponds to one example.

As used herein, the term "C-terminus" refers to a carboxy terminus of the peptide, and for the purpose of the present invention, the term refers to a position that can be linked to a linker. For example, although not limited, the C-terminus may include not only the amino acid residue at the outermost end of the C-terminus but also amino acid residues near the C-terminus, and specifically the 1^{st} to 20^{th} amino acid residues from the outermost end.

The above-described conjugate may have an increase in the duration of efficacy compared with those having X which is not modified with F, and such a conjugate is not only in the above-described form, but also in the form encapsulated in biodegradable nanoparticles.

Unless specified otherwise herein, the contents in the detailed description and claims with respect to the "peptide" according to the present invention or a "conjugate" in which this peptide is covalently linked to a biocompatible material through a covalent linkage are applied to the form of not only the corresponding peptide or conjugate but also a salt of the corresponding peptide or conjugate (e.g., a pharmaceutically acceptable salt of the peptide), or a solvate thereof. Therefore, although described as "peptide" or "conjugate" herein, the corresponding described contents are equally applied to a specific salt thereof, a specific solvate thereof, and a specific solvate of the specific salt thereof. These salts may be in the form in which, for example, any pharmaceutically acceptable salt is used. The type of the salt is not particularly limited. However, the salt is preferably in the form that is safe and effective to a subject, e.g., a mammal, but is not particularly limited thereto.

The type of the salt is not particularly limited. However, the salt is preferably in the form that is safe and effective to a subject, e.g., a mammal, but is not particularly limited thereto.

The term "pharmaceutically acceptable" refers to a substance that can be effectively used for a desired purpose without causing excessive toxicity, irritation, allergic responses, and the like within the range of the medical and pharmaceutical decision.

As used herein, the term "pharmaceutically acceptable salt" refers to a salt derived from pharmaceutically acceptable inorganic acids, organic acids, or bases. Examples of appropriate acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from appropriate bases may include alkali metals such as sodium and potassium, alkali earth metals such as magnesium, ammonium, and the like.

As used herein, the term "solvate" refers to a complex formed between the peptide according to the present invention or a salt thereof and a solvent molecule.

The composition according to the present invention may contain a peptide (triple agonist) or a conjugate thereof, and specifically may contain a pharmaceutically effective amount of a peptide or a conjugate thereof. The composition of the present invention may further contain a pharmaceutically acceptable carrier. The composition of the present invention may have uses for lowering blood pressure. Alternatively, the composition of the present invention may have uses for prevention, treatment, or alleviation of hypertension, but is not limited thereto.

As used herein, the term "pharmaceutical acceptable" refers to a sufficient amount to show therapeutic effects without causing side effects, and the amount may be easily determined by a person skilled in the art according to factors that are well known in the medical field, including the type of disease, patient's age, body weight, health condition, and sex, the sensitivity of a patient to drugs, the route of administration, the method of administration, the number of times of administration, the period of treatment, drugs used in combination or at the same time, and the like.

Regarding the pharmaceutically acceptable carrier, a binder, a lubricant, a disintegrant, a solubilizer, a dispersant, a stabilizer, a suspending agent, a coloring agent, a flavoring agent, and the like may be used for oral administration; a buffer, a preservative, an analgesic, a solubilizer, an isotonic agent, a stabilizer, and the like may be used in combination for injectable preparations; and a base, an excipient, a lubricant, a preservative, and the like may be used for topical administration. The formulations of the pharmaceutical composition of the present invention may be prepared in various manners by mixing with the pharmaceutically acceptable carriers described above. For example, for oral administration, the pharmaceutical composition may be formulated in the form of a tablet, a troche, a capsule, an elixir, a suspension, a syrup, a wafer, or the like; and for injections, the pharmaceutical composition may be formulated in the form of a unit-dosing ampoule or a multidosing form. Besides, the pharmaceutical composition may also be formulated into a solution, a suspension, a tablet, pills, a capsule, a sustained-release preparation, and the like.

Meanwhile, examples of a carrier, an excipient, and a diluent suitable for the formulation may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, and the like. In addition, a filler, an anti-coagulant, a lubricant, a wetting agent, a flavor, an emulsifier, a preservative, and the like may further be included.

The composition of the present invention may have any one formulation selected from the group consisting of a tablet, a pill, a powder, granules, a capsule, a suspension, a liquid preparation for internal use, an emulsion, a syrup, a sterile aqueous solution, a non-aqueous solvent, a lyophilized preparation, and a suppository.

In addition, the conjugate may be used by mixing with various carriers approved as drugs, such as physiological saline or organic solvents, and for increasing stability or absorptivity, carbohydrates such as glucose, sucrose, or dextran, antioxidants such as ascorbic acid or glutathione, chelating agents, lowmolecular weight proteins, or other stabilizers may be used as medical agents.

The dose and frequency of the composition of the present invention are determined according to the type of drug as an active ingredient, along with various factors, such as a disease to be treated, a route of administration, patient's age, sex, and body weight, and severity of a disease. Specifically, the composition of the present invention may contain a pharmaceutically effective amount of the peptide or the conjugate thereof, but is not limited thereto.

Containing the peptide or a long-acting conjugate thereof at a pharmaceutically effective amount refers to a level at which desired pharmaceutical activity (e.g., lowing blood pressure) can be obtained by the peptide or a long-acting conjugate thereof, and may also refer to a level at which toxicities or side effects do not occur or occur at an insignificant level or a pharmaceutically acceptable level in a subject to be administered, but the level is not limited thereto. Such a pharmaceutically effective amount may be determined by comprehensively considering the frequency of administration, patient, formulations, and the like.

Although not particularly limited, the pharmaceutical composition of the present invention may contain the ingredient (active ingredient) at an amount of 0.01% (w/v) to 99% (w/v).

The total effective dose of the composition of the present invention may be administered to a patient in a single dose, or may be administered in multiple doses according to a fractionated treatment protocol for a long period of time. In the composition of the present invention, the content of an active ingredient may vary according to the severity of a disease. Specifically, the preferable total daily dose of the peptide or the conjugate thereof of the present invention may be about 0.0001 mg to 500 mg per 1 kg of body weight of a patient. However, the dose of the peptide or the conjugate thereof is determined considering various factors including patient's age, body weight, health condition, sex, severity of a disease, a diet, and an excretion rate, in addition to the route of administration and the frequency of treatment of the pharmaceutical composition. Therefore, considering these, a person skilled in the art may easily determine an appropriate effective dose according to particular uses of the composition of the present invention. The pharmaceutical composition according to the present invention is not particularly limited to the formation, route of administration, and method of administration, as long as the pharmaceutical composition shows the effects according to the present invention.

For example, the composition of the present invention may be administered once a week, once every 2 weeks, once every 4 weeks, or once a month, but is not limited thereto.

The above description can be applied to other embodiments or aspects of the present invention, but is not limited thereto.

In accordance with another aspect of the present invention, there is provided a method for lowering blood pressure, the method including administering a peptide, a conjugate thereof, or a composition comprising the peptide or the conjugate thereof to a subject.

The peptide, the conjugate thereof, the composition, and the blood pressure lowing are as described above.

In the present invention, the subject is a subject for which blood pressure lowering is beneficial, and means a mammal including a rat, livestock, and the like, including a human being, but any subject for which a blood pressure lowering effect by the peptide or the conjugate thereof, or the composition containing the peptide or the conjugate thereof of the present invention is beneficial is included without limitation. In particular, the subject may have metabolic syndrome or a liver disease, but is not limited thereto. In an embodiment, the subject may have obesity, fatty liver, or obesity and fatty liver, but is not limited thereto.

As used herein, the term "administration" refers to the introduction of a predetermined substance to a patient (subject) by any suitable method, and the route of administration of the peptide, the conjugate thereof, or composition, but is not particularly limited, may be any general route through which the peptide, the conjugate thereof, or the composition can reach a target *in vivo,* for example, intraperitoneal administration, intravenous administration, intramuscular administration, subcutaneous administration, intradermal administration, oral administration, topical administration, intranasal administration, intrapulmonary administration, intrarectal administration, or the like.

In an embodiment, the administration may be parenteral administration or subcutaneous administration, but is not limited thereto.

The peptide, the conjugate thereof, or the composition may be formulated into a single dosage form suitable for the patient's body, and specifically may be formulated into a preparation useful for the administration of a protein drug, and may be administered by an administration method commonly used in the art through an oral or parenteral route, such as through skin, intravenous, intramuscular, intraarterial, intramedullary, intrathecal, intraventricular, pulmonary, transdermal, subcutaneous, intraperitoneal, intranasal, intra-gastric, topical, sublingual, vaginal, or rectal route, but is not limited thereto.

Although not limited, the peptide, the conjugate thereof, or the composition containing the peptide or the conjugate thereof of the present invention may be administered once a week, once every 2 weeks, once every 4 weeks, or once a month, but is not limited thereto. In an embodiment, the composition may be parenterally administered once a week, but is not limited thereto.

In a still embodiment, the composition is administered once a week for 4 weeks, and the dose thereof may be increased and administered one e seek, but is not limited thereto.

The method of the present invention may include administering the composition containing the peptide or the conjugate thereof at a pharmaceutically effective amount. The appropriate total daily dose of the composition may be determined by a practitioner within the range of correct medical judgement, and the composition may be administered once or several times in divided doses. For the purpose of the present invention, the specific therapeutically effective dose for a specific patient may be preferably applied differently depending on not only various factors including the type and degree of response to be achieved, a specific composition according to whether other agents are occasionally used therewith or not, the patient's age, body weight, general health condition, sex and a diet, the time of administration, the route of administration, the secretion rate of the composition, the duration of treatment, drugs used in combination or concurrently with the specific composition, but also similar factors well-known in the medicinal art.

In an embodiment, the peptide or the long-acting conjugate may be parenterally administered at 0.1 mg to 15 mg once a week, but is not limited thereto.

The weekly dose of the peptide or the long-acting conjugate of the present invention may be 0.1 mg to 15 mg, 0.1 mg to 14 mg, 1 mg to 14 mg, 1 mg to 13 mg, 1 mg to 10 mg, 1 mg to 9 mg, 1.5 mg to 10 mg, 1.5 mg to 3 mg, 1.5 mg to 2.5 mg, 2 mg, 2 mg to 10 mg, 2.5 mg to 5 mg, 2.5 mg to 4.5 mg, 3 mg to 4.5 mg, 4 mg, 3.5 mg to 10 mg, 4 mg to 9.5 mg, 4.5 mg to 8.5 mg, 5 mg to 7 mg, 5.5 mg to 7 mg, 5.5 mg to 6.5 mg, 6 mg, 6 mg to 10 mg, 6 mg to 9 mg, 6.5 mg to 9.5 mg, 7 mg to 9 mg, 7.5 mg to 9.5 mg, 7.5 mg to 8.5 mg, or 8 mg, but is not limited thereto.

The weekly dose of the long-acting conjugate of the present invention may be 0.1 mg to 15 mg, 0.1 mg to 14 mg, 1 mg to 14 mg, 1 mg to 13 mg, 1 mg to 10 mg, 1 mg to 9 mg, 1.5 mg to 10 mg, 1.5 mg to 3 mg, 1.5 mg to 2.5 mg, 2 mg, 2 mg to 10 mg, 2.5 mg to 5 mg, 2.5 mg to 4.5 mg, 3 mg to 4.5 mg, 4 mg, 3.5 mg to 10 mg, 4 mg to 9.5 mg, 4.5 mg to 8.5 mg, 5 mg to 7 mg, 5.5 mg to 7 mg, 5.5 mg to 6.5 mg, 6 mg, 6 mg to 10 mg, 6 mg to 9 mg, 6.5 mg to 9.5 mg, 7 mg to 9 mg, 7.5 mg to 9.5 mg, 7.5 mg to 8.5 mg, or 8 mg, but is not limited thereto.

In the composition according to any one of the previous embodiments, the peptide or the long-acting conjugate may be parenterally administered at 1 mg to 10 mg once a week for 4 weeks, but is not limited thereto.

In the composition according to any one of the previous embodiments, the long-acting conjugate may be parenterally administered at 1 mg to 10 mg once a week for 4 weeks, but is not limited thereto.

In the composition according to any one of the previous embodiments, the peptide or the long-acting conjugate may be parenterally administered at 2 mg, 4 mg, 6 mg, or 8 mg once a week for 4 weeks, but is not limited thereto.

In the composition according to any one of the previous embodiments, the long-acting conjugate may be parenterally administered at 2 mg, 4 mg, 6 mg, or 8 mg once a week for 4 weeks, but is not limited thereto.

In accordance with still another aspect to implement the present invention, there is provided use of a peptide or a conjugate thereof for lowering blood pressure.

In accordance with still another aspect to implement the present invention, there is provided use of a peptide or a conjugate thereof, for the preparation of a medicament for use in lowering blood pressure.

The peptide, the conjugate thereof, the composition, and the blood pressure lowing are as described above.

Hereinafter, the present invention will be described in detail with reference to the following examples. However, the following examples are merely for illustrating the present invention and are not intended to limit the scope of the present invention.

### Example 1: Preparation of triple agonists

Triple agonists exhibiting activities for all of GLP-1, GIP, and glucagon receptors were prepared, and amino acid sequences thereof are shown in Table 1 below.

**TABLE 1**

| SEQ ID NO | Sequence | Information |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P P P S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | HXQGTFTSDVSKYLDGQAAQEFVAWLLAG GGPSSGAPPPSC | |
| 11 | CAGEGTFTSDLSKYLDSRRQQLFVQWLKA | |
| | G G P S S G A P P P S H G | |
| 12 | CAGEGTFISDLSKYMDEQAVQLFVEWLMA GGPSSGAPPPSHG | |
| 13 | CAGEGTFISDYSIQLDEIAVQDFVEWLLAQ KPSSGAPPPSHG | |
| 14 | CA G Q G T F T S D Y S I Q L D E I A V R D F V E W L K N G G P S S G A P P P S H G | |
| 15 | CA G Q G T F T S D L S K Q M D E E A V R L F I E W L K N G G P S S G A P P P S H G | |
| 16 | CA G Q G T F T S D L S K Q M D S E A Q Q L F I E W L K N G G P S S G A P P P S H G | |
| 17 | CAGQGTFTSDLSKQMDEERAREFIEWLLA QKPSSGAPPPSHG | |
| 18 | CA G Q G T F T S D L S K Q M D S E R A R E F I E W L K N T G P S S G A P P P S H G | |
| 19 | CA G Q G T F T S D L S I Q Y D S E H Q R D F I E W L K D T G P S S G A P P P S H G | |
| 20 | CA G Q G T F T S D L S I Q Y E E E A Q Q D F V E W L K D T G P S S G A P P P S H G | |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G P S S G A P P P S | Ring formation |
| 25 | HXQGTFTSDCSKYLDERAAQDFVQWLLDG GPSSGAPPPS | |
| 26 | HXQGTFTSDCSKYLDSRAAQDFVQWLLDG GPSSGAPPPS | |
| 27 | H X Q G T F T S D Y S K Y L D E R A C Q D F V Q W L L D Q | |
| | G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | Ring formation |
| 30 | H X Q G T F T S D Y S K Y L D E K A Q K E F V Q W L L D T C | Ring formation |
| 31 | HXQGTFTSDYSKYLD**E**KAC**K**EFVQWLLAQ | Ring formation |
| 32 | H X Q G T F T S D Y S K Y L D E K A C K D F V Q W L L D G G P S S G A P P P S | Ring formation |
| 33 | H X Q G T F T S D Y S I A M D E I H Q K D F V N W L L A Q K C | Ring formation |
| 34 | H X Q G T F T S D Y S K Y L D E K R Q K E F V N W L L A Q K C | Ring formation |
| 35 | H X Q G T F T S D Y S I A M D E I H Q K D F V N W L L N T K C | Ring formation |
| 36 | H X Q G T F T S D Y S K Y L C E K R Q K E F V Q W L L N G G P S S G A P P P S G | Ring formation |
| 37 | H X Q G T F T S D Y S K Y L D E C R Q K E F V Q W L L N G G P S S G A P P P S G | Ring formation |
| 38 | CAXQGTFTSDKSSYLDERAAQDFVQWLLD GGPSSGAPPPSS | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H | Ring |
| | HCSSGQPPPS | formation |
| 44 | HGQGTFTSDCSKQLDGQAAQEFVAWLLAG GPSSGAPPPS | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | CAXQGTFTSDYSICMDEIHQKDFVNWLLNT K | Ring formation |
| 50 | H X Q G T F T S D Y S K Y L D E K R A K E F V Q W L L D H H P S S G Q P P P S C | Ring formation |
| 51 | H X Q G T F T S D Y S K Y L D E K R Q K E F V Q W L L N T C | Ring formation |
| 52 | H X Q G T F T S D Y S K Y L D E K R Q K E F V Q W L L D T C | Ring formation |
| 53 | H X E G T F T S D Y S I A M D E I H Q K D F V N W L L A Q C | Ring formation |
| 54 | HXEGTFTSDYSIAMD**E**IHQ**K**DFVDWLLAEC | Ring formation |
| 55 | H X Q G T F T S D Y S I A M D E I H Q K D F V N W L L A Q C | Ring formation |
| 56 | H X Q G T F T S D Y S K Y L D E K R Q K E F V N W L L A Q C | Ring formation |
| 57 | H X Q G T F T S D Y S I A M D E I H Q K D F V N W L L N T C | Ring formation |
| 58 | H X Q G T F T S D Y S K Y L D E K R Q K E F V Q W L L N T K C | Ring formation |
| 59 | CAXQGTFTSDYSICMDEKHQKDFVNWLLN | Ring |
| | T K | formation |
| 60 | CAXQGTFTSDYSIAMDEKHCKDFVNWLLN TK | Ring formation |
| 61 | CAXQGTFTSDYSIAMDEIACKDFVNWLLNT K | Ring formation |
| 62 | CAXQGTFTSDKSKYLDERAAQDFVQWLLD GGPSSGAPPPS | |
| 63 | CA X Q G T F T S D C S K Y L D E R A A Q D F V Q W L L D G G P S S G A P P P S | |
| 64 | Y X Q G T F T S D Y S K Y L D E C A A K E F V Q W L L D H H P S S G Q P P P S | Ring formation |
| 65 | H X Q G T F T S D Y S K C L D E K R A K E F V Q W L L D H H P S S G Q P P P S | Ring formation |
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | CAXQGTFTSDYSKYLDECRAKEFVQWLLD HHPSSGQPPPS | Ring formation |
| 74 | CAXQGTFTSDYSKCLDEKRAKEFVQWLLD HHPSSGQPPPS | Ring formation |
| 75 | Y X Q G T F T S D Y S K Y L D E K A A K E F V Q W L L D H | Ring |
| | H P S S G Q P P P S C | formation |
| 76 | Y X Q G T F T S D Y S K Y L D E K R A K D F V Q W L L D H H P S S G Q P P P S C | Ring formation |
| 77 | Y X Q G T F T S D Y S K Y L D E K A A K D F V Q W L L D H H P S S G Q P P P S C | Ring formation |
| 78 | H X Q G T F T S D Y S K Y L D E K R Q K E F V Q W L L D T K C | Ring formation |
| 79 | H X E G T F T S D Y S I A M D E I H Q K D F V N W L L A Q K C | Ring formation |
| 80 | H X E G T F T S D Y S I A M D E I H Q K D F V D W L L A E K C | Ring formation |
| 81 | CAXQGTFTSDYSKYLDEKRQKEFVQWLLN TC | Ring formation |
| 82 | C A X Q G T F T S D Y S K Y L D E K R Q K E F V Q W L L D T C | Ring formation |
| 83 | CA X E G T F T S D Y S I A M D E I H Q K D F V N W L L A Q C | Ring formation |
| 84 | CA X E G T F T S D Y S I A M D E I H Q K D F V D W L L A E C | Ring formation |
| 85 | CA X Q G T F T S D Y S I A M D E I H Q K D F V N W L L A Q C | Ring formation |
| 86 | CA X Q G T F T S D Y S K Y L D E K R Q K E F V N W L L A Q C | Ring formation |
| 87 | CA X Q G T F T S D Y S I A M D E I H Q K D F V N W L L N T C | Ring formation |
| 88 | CAXQGTFTSDYSKYLDEKRQKEFVQWLLN TKC | Ring formation |
| 89 | CAXQGTFTSDYSKYLDEKRQKEFVQWLLD TKC | Ring formation |
| 90 | CAXEGTFTSDYSIAMDEIHQKDFVNWLLAQ KC | Ring formation |
| 91 | CAXEGTFTSDYSIAMDEIHQKDFVDWLLAE | Ring |
| | K C | formation |
| 92 | CA X Q G T F T S D Y S I A M D E I H Q K D F V N W L L A Q K C | Ring formation |
| 93 | CA X Q G T F T S D Y S K Y L D E K R Q K E F V N W L L A Q K C | Ring formation |
| 94 | CA X Q G T F T S D Y S I A M D E I H Q K D F V N W L L N T K C | Ring formation |
| 95 | Y X Q G T F T S D Y S K Y L D E K R A K E F V Q W L L C H H P S S G Q P P P S | Ring formation |
| 96 | Y X Q G T F T S D Y S K Y L D E K R A K E F V Q W L L D H C P S S G Q P P P S | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | Y X Q G T F T S D Y S K A L D E K A A K D F V N W L L D Q H P S S G Q P P P S C | Ring formation |

In the sequences shown in Table 1, the amino acid marked with X represents 2-aminoisobutyric acid (Aib), a non-native amino acid, and the underlined amino acids indicate the formation of a ring between the bold and underlined amino acids. In Table 1, CA represents 4-imidazoacetyl, and Y represents tyrosine.

### Example 2: Preparation of long-acting conjugates of triple agonists

To PEGylate cysteine residues of the triple agonists (SEQ ID NOS: 21, 22, 42, 43, 50, 77, and 96) of Example 1 with PEG (10 kDa) having a maleimide group at one end and an aldehyde group at the other end, that is, maleimide-PEG-aldehyde (10 kDa, NOF, Japan), the triple agonists and the maleimide-PEG-aldehyde were reacted at a molar ratio of 1:1-3, a protein concentration of 1 mg/mL to 5 mg/mL, and a low temperature, for 0.5 to 3 hours. The reaction was conducted under an environment in which 20-60% isopropanol was added to 50 mM Tris buffer (pH 7.5). Upon completion of the reaction, the reaction solutions were applied to SP Sepharose HP (GE Healthcare, USA) to purify triple agonists having mono-PEGylated cysteines.

Then, the purified mono-PEGylated triple agonists and an immunoglobulin Fc (the homodimer of SEQ ID NO: 123) were reacted at a molar ratio of 1:1-5, a protein concentration of 10 mg/mL to 50 mg/mL, and 4°C to 8°C for 12 to 18 hours. The reaction was conducted under an environment in which 10 mM to 50 mM sodium cyanoborohydride as a reducing agent and 10% to 30% isopropanol were added to a 100 mM potassium phosphate butter (pH 6.0). Upon completion of the reaction, the reaction solutions were applied to the butyl sepharose FF purification column (GE Healthcare, USA) and Source ISO purification column (GE Healthcare, USA) to purify conjugates containing triple agonists and immunoglobulin Fc. The purified long-acting conjugates have a structure in which a triple agonist peptide, a polyethylene glycol (PEG) linker, and an Fc dimer are covalently linked at a molar ratio of 1:1:1, wherein the PEG linker is linked to only one of the two polypeptide chains of the Fc dimer.

In the immunoglobulin Fc, two monomers having the amino acid sequence of SEQ ID NO: 123 (consisting of 221 amino acids) form a homodimer through a disulfide bond between cysteines, which correspond to the amino acid at position 3 of each monomer, wherein the monomers of the homodimer, independently, form an intra-disulfide bond between the cysteines at positions 35 and 95 and an intra-disulfide bond between the cysteines at positions 141 and 199, respectively.

The purity of the conjugates that was analyzed by reverse-phase chromatography, size-exclusion chromatography, and ion-exchange chromatography after the preparation was 95% or higher.

In particular, the conjugate in which the triple agonist of SEQ ID NO: 21 and the immunoglobulin Fc were linked via the PEG linker was named "the conjugate including SEQ ID NO: 21 and immunoglobulin Fc" or "the long-acting conjugate of SEQ ID NO: 21", and these may be used interchangeably herein.

In particular, the conjugate in which the triple agonist of SEQ ID NO: 22 and the immunoglobulin Fc were linked via the PEG linker was named "the conjugate including SEQ ID NO: 22 and immunoglobulin Fc" or "the long-acting conjugate of SEQ ID NO: 22", and these may be used interchangeably herein.

In particular, the conjugate in which the triple agonist of SEQ ID NO: 42 and the immunoglobulin Fc were linked via the PEG linker was named "the conjugate including SEQ ID NO: 42 and immunoglobulin Fc" or "the long-acting conjugate of SEQ ID NO: 42", and these may be used interchangeably herein.

In particular, the conjugate in which the triple agonist of SEQ ID NO: 43 and the immunoglobulin Fc were linked via the PEG linker was named "the conjugate including SEQ ID NO: 43 and immunoglobulin Fc" or "the long-acting conjugate of SEQ ID NO: 43", and these may be used interchangeably herein.

In particular, the conjugate in which the triple agonist of SEQ ID NO: 50 and the immunoglobulin Fc were linked via the PEG linker was named "the conjugate including SEQ ID NO: 50 and immunoglobulin Fc" or "the long-acting conjugate of SEQ ID NO: 50", and these may be used interchangeably herein.

In particular, the conjugate in which the triple agonist of SEQ ID NO: 77 and the immunoglobulin Fc were linked via the PEG linker was named "the conjugate including SEQ ID NO: 77 and immunoglobulin Fc" or "the long-acting conjugate of SEQ ID NO: 77", and these may be used interchangeably herein.

In particular, the conjugate in which the triple agonist of SEQ ID NO: 96 and the immunoglobulin Fc were linked via the PEG linker was named "the conjugate including SEQ ID NO: 96 and immunoglobulin Fc" or "the long-acting conjugate of SEQ ID NO: 96", and these may be used interchangeably herein.

### Experimental Example 1: In vitro activities of triple agonists and long-acting conjugates thereof

To determine the activities of the triple agonists and the long-acting conjugates thereof prepared in Examples 1 and 2, the cell activity was measured *in vitro* by using cell lines in which GLP-1 receptor, glucagon (GCG) receptor, and GIP receptor were transformed, respectively.

The cell lines were obtained by transforming Chinese hamster ovary (CHO) cells to express human GLP-1 receptor, human GCG receptor, and human GIP receptor, respectively, and are suitable for the measurement of the activities of GLP-1, GCG, and GIP. Therefore, the activities for the respective parts were measured using the transformed cell lines, respectively.

For the measurement of the GLP-1 activity of the triple agonists and the long-acting conjugates prepared in Examples 1 and 2, human GLP-1 was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GLP-1 receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysate, upon completion of the reaction, was applied to the LANCE cAMP kit (PerkinElmer, USA) to calculate EC₅₀ values through accumulated cAMP, and then the values were compared with each other. The relative titers compared to human GLP-1 are shown in Tables 2 and 3 below.

For the measurement of the GCG activity of the triple agonists and the long-acting conjugates prepared in Examples 1 and 2, human GCG was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GCG receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysate, upon completion of the reaction, was applied to the LANCE cAMP kit (PerkinElmer, USA) to calculate EC₅₀ values through accumulated cAMP, and then the values were compared with each other. The relative titers compared to human GCG are shown in Tables 2 and 3 below.

For the measurement of the GIP activity of the triple agonists and the long-acting conjugates prepared in Examples 1 and 2, human GIP was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GIP receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysate, upon completion of the reaction, was applied to the LANCE cAMP kit (PerkinElmer, USA) to calculate EC₅₀ values through accumulated cAMP, and then the values were compared with each other. The relative titers compared to human GIP are shown in Tables 2 and 3 below.

**TABLE 2**

| Relative titer ratio of triple agonists | | | |
|---|---|---|---|
| | *In vitro* activity compared with native peptide (%) | | |
| SEQ ID NO | *vs.* GLP-1 | *vs.* Glucagon | *vs.* GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**TABLE 3**

| Relative titer ratio of long-acting conjugates of triple agonists | | | |
|---|---|---|---|
| Long-acting conjugate | *In vitro* activity compared with native peptide (%) | | |
| | *vs*. GLP-1 | *vs*. Glucagon | *vs*. GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The triple agonists and the long-acting conjugates thereof have a function as a triple agonist capable of activating all of GLP-1 receptor, GIP receptor, and glucagon receptor.

### Experimental Example 2: Blood vessel dilating function of long-acting agonist of triple agonist confirmed in human umbilical vein endothelial cells (HUVECs)

To evaluate the efficacy of the long-acting conjugate of SEQ ID NO: 42 prepared in Examples 1 and 2 on a cardiovascular disease, the phosphorylation of endothelial nitric oxide synthase (eNOS) used as an *in vitro* blood vessel dilation-related indicator was measured by using human umbilical vein endothelial cells (HUVECs), thereby investigating the expression and activity levels of the endothelial nitric oxide synthase.

To evaluate the blood vessel dilating function of a long-acting conjugate of the triple agonist prepared in Examples 1 and 2, human umbilical vein endothelial cells were cultured so that efficacy evaluation could be performed on a control group treated with only vehicle for the long-acting conjugate of SEQ ID NO: 42, a group treated with the long-acting conjugate of SEQ ID NO: 42 (10 µM), and a group treated with liraglutide (50 µM). Each of the substances was added to the cultured cells, and after 1 hour, the cells were lysed using a cell lysis buffer, and then Western blot was used to determine the phosphorylation level of endothelial nitric oxide synthase (eNOS) in the cells of each group, followed by comparison with each other (FIG. 1)

As a result, the long-acting conjugate of the triple agonist according to the present invention showed a significant increase in the phosphorylation of endothelial nitric oxide synthase in the human umbilical vein endothelial cells, compared with the control group treated only with vehicle for the long-acting conjugate of the triple agonist and the comparison group treated with liraglutide (50 µM). These results indicate the blood vessel dilating function of the long-acting conjugate of the triple agonist, suggesting that the long-acting conjugate of the triple agonist can be used as a therapeutic substance for a disease.

The phosphorylation levels of endothelial nitric oxide synthase were corrected through beta-actin, and statistical analysis was performed to compare between the control group and the test group by one-way ANOVA.

### Experimental Example 3: Blood pressure lowering effect of long-acting conjugate of triple agonist

To directly investigate the blood pressure lowering effect of the long-acting conjugate of SEQ ID NO: 42 prepared in Examples 1 and 2, the following test was conducted.

The long-acting conjugate of SEQ ID NO: 42 was repeatedly subcutaneously administered to humans at 0.01 mg/kg to 0.08 mg/kg, and the blood pressure lowering effect was investigated 12 weeks after administration compared with before administration.

Specifically, a sterilized, colorless solution containing a conjugate in which only an immunoglobulin Fc region and PEG were linked (not containing the triple agonist of SEQ ID NO: 42) was administered to a control test group (placebo), and different doses (0.01 mg/kg, 0.02 mg/kg, 0.04 mg/kg, 0.06 mg/kg and 0.08 mg/kg) were administered to the test groups. Then, after the administration, the blood pressure was continuously measured (24-hour ABPM, ambulatory blood pressure monitoring) to determine heart rate (HR), systolic blood pressure (SBP), diastolic blood pressure (DBP), and rate pressure product (RPP).

As a result, the humans administered with the long-acting conjugate of SEQ ID NO: 42 showed reductions in the systolic blood pressure (SBP), diastolic blood pressure, compared with the control group. It was therefore confirmed that the long-acting conjugate of the triple agonist had a blood pressure lowering effect (Table 4).

**TABLE 4**

| | Control group (placebo, N=15) | Long-acting conjugate of SEQ ID NO: 42 0.01 mg/kg (N=9) | Long-acting conjugate of SEQ ID NO: 42 0.02 mg/kg (N=10) | Long-acting conjugate of SEQ ID NO: 42 0.04 mg/kg (N=9) | Long-acting conjugate of SEQ ID NO: 42 0.06 mg/kg (N=9) | Long-acting conjugate of SEQ ID NO: 42 0.08 mg/kg (N=7) |
|---|---|---|---|---|---|---|
| Systolic blood pressure (SBP; mmHg) | 1.2±8.1 | -1.017.2 | 1.1±7.9 | -10.419.2 | -8.019.2 | -12.1±8.0 |
| Diastolic blood pressure (DBP; mmHg) | -0.3±4.5 | -1.217.4 | -0.814.2 | -3.615.0 | -2.1±6.3 | -5.615.8 |

The above results confirmed that the blood vessel dilation was induced in the humans by the long-acting conjugate of the triple agonist, leading to a blood pressure effect, suggesting that the triple agonists of the present invention can be used as therapeutic agents for various diseases requiring blood pressure lowering.

While the present invention has been described with reference to the particular illustrative embodiments, a person skilled in the art to which the present invention pertains can understand that the present invention may be embodied in other specific forms without departing from the technical spirit or essential characteristics thereof. In this regard, the embodiments described above should be understood to be illustrative rather than restrictive in every respect. The scope of the invention should be construed as the meaning and scope of the appended claims rather than the detailed description, and all changes or variations derived from the equivalent concepts fall within the scope of the present invention.

## Claims

1. A pharmaceutical composition for lowering blood pressure, the composition comprising:
a pharmaceutically acceptable excipient; and
a peptide including an amino acid sequence of any one of SEQ ID NOS: 1 to 102.

2. The composition of claim 1, wherein the peptide is in the form of a long-acting conjugate, and the long-acting conjugate is represented by Formula 1 below:
[Formula 1] X-L-F
wherein X represents a peptide including an amino sequence of any one of SEQ ID NOS: 1 to 102;
L represents a linker containing ethylene glycol repeating units;
F represents an immunoglobulin Fc region; and
"-" represents covalent linkages between X and L and between L and F, respectively.

3. The composition of claim 1 or 2, wherein the composition shows a blood pressure lowering effect through blood vessel dilation in a subject.

4. The composition of claim 1 or 2, wherein the composition increases the phosphorylation of endothelial nitric oxide synthase (eNOS).

5. The composition of claim 3, wherein the subject has a metabolic syndrome or liver disease.

6. The composition of claim 1 or 2, wherein the peptide is C-terminally amidated.

7. The composition of claim 1 or 2, wherein the peptide has a ring formed between amino acid residues.

8. The composition of claim 2, wherein the immunoglobulin Fc region is aglycosylated.

9. The composition of claim 2, wherein the immunoglobulin Fc region is an IgG4 Fc region.

10. The composition of claim 2, wherein the immunoglobulin Fc region is a dimer consisting of two polypeptide chains, and one end of L is linked to only one of the two polypeptide chains.

11. The composition of claim 2, wherein in the conjugate, L is linked to F and X by covalent linkages formed by reacting one end of L with an amine group or thiol group of F and reacting the other end of L with an amine group or thiol group of X, respectively.

12. The composition of claim 2, wherein L is polyethylene glycol.

13. The composition of claim 2, wherein the formula weight of a moiety of the ethylene glycol repeating units in L is in the range of 1 kDa to 100 kDa.

14. The composition of claim 5, wherein the metabolic syndrome is selected from the group consisting of impaired glucose tolerance, hypercholesterolemia, dyslipidemia, obesity, diabetes, hypertension, non-alcoholic steatohepatitis (NASH), atherosclerosis caused by dyslipidemia, atherosclerosis, arteriosclerosis, coronary heart disease, and stroke.

15. The composition of claim 5, wherein the liver disease is selected from the group consisting of alcoholic liver disease, non-alcoholic liver disease, metabolic liver disease, liver fibrosis, liver cirrhosis, fatty liver, hepatitis, viral liver disease, hepatitis, hepatotoxicity, cholestasis, fatty liver, cirrhosis, liver ischemia, liver abscess, hepatic coma, liver atrophy, liver failure, cholestatic liver disease, primary biliary cirrhosis, primary sclerosing cholangitis, and liver cancer.

16. The composition of claim 3, wherein the subject has i) a metabolic disease, ii) a liver disease, or iii) a metabolic disease and a liver disease, accompanied by a hypertensive disease.

17. The composition of claim 3, wherein the subject has non-alcoholic fatty liver disease accompanied by a metabolic disease.

18. The composition of claim 3, wherein the subject has non-alcoholic steatohepatitis (NASH) accompanied by a metabolic disease.

19. The composition of claim 3, wherein the subject has at least one metabolic disease risk factor.

20. The composition of claim 14, wherein the composition has a blood pressure lowering effect in a subject having obesity.

21. The composition of claim 15, wherein the composition has a blood pressure lowering effect in a subject having fatty liver.

22. The composition of claim 5, wherein the composition has a blood pressure lowering effect in a subject having obesity and fatty liver.

23. The composition of claim 3, wherein the composition is used for treating non-alcoholic fatty liver disease in a subject at risk of developing obesity or a metabolic disease.

24. The composition of claim 3, wherein the composition is used for treating non-alcoholic steatohepatitis (NASH) in a subject at risk of developing obesity or a metabolic disease.

25. The composition of claim 1 or 2, wherein the peptide or the long-acting conjugate is parenterally administered once a week.

26. The composition of claim 1 or 2, wherein the peptide or the long-acting conjugate is subcutaneously administered.
